# EUROPÄISCHE PATENTANMELDUNG

(11) **EP 4 691 350 A2**
(43) Veröffentlichungstag der Anmeldung: **11.02.2026**
(21) Anmeldenummer: 25225070.9
(22) Anmeldetag: 18.02.2022
(51) Int. Cl.: A61B 5/00

(54) **KALIBRIERSYSTEM FÜR EINEN ÖSOPHAGUSKATHETER MIT BALLONSONDE ZUR BESTIMMUNG EINES ÖSOPHAGEALEN DRUCKS**

(30) Priorität: 02.03.2021 DE 102021104993
(62) Teilanmeldung aus: 22707677.5
(71) Anmelder: Hamilton Medical AG, 7402 Bonaduz (CH)
(72) Erfinder: Schwenninger, David, 7012 Felsberg (CH)
(74) Vertreter: Schmitt-Nilson Schraud Waibel Wohlfrom Patentanwälte Partnerschaft mbB

(57) **Zusammenfassung**

Die Erfindung betrifft ein Kalibriersystem (60) zur automatisierten Einstellung einer betriebsgemäßen Befüllung eines in den Ösophagus (34) einführbaren Ösophaguskatheters (48) mit Ballonsonde (46) zur Bestimmung eines ösophagealen Drucks (Peso), insbesondere für eine Beatmungsvorrichtung (10), umfassend eine Anordnung zum Befüllen der Ballonsonde (46) mit einem Messfluid nach Platzieren der Ballonsonde (46) im Ösophagus (34), einen Drucksensor zum Erfassen des in der Ballonsonde (46) herrschenden ösophagealen Drucks (Peso), sowie eine Kalibriersteuerung (60) die derart ausgebildet ist, dass sie die Menge an Messfluid in der Ballonsonde (46) schrittweise verändert, wobei die Kalib-riersteuerung (60) bei jeder der auf diese Weise schrittweise als Messpunkte eingestellten Mengen an Messfluid in der Ballonsonde (46) einen von dem Drucksensor erfassten ösophagealen Druck (Peso) aufnimmt und der jeweils eingestellten Menge an Messfluid in der Ballonsonde (46) zuordnet. Die Kalibriersteuerung (60) ist derart ausgebildet, dass sie zum Anfahren der jeweiligen Messpunkte die Menge an Messfluid ausgehend von einem Startwert bis zum Erreichen eines Endwertes in mindestens zwei Schritten in monotoner Weise verändert.

## Beschreibung

Die vorliegende Erfindung betrifft ein Kalibriersystem für einen Ösophaguskatheter mit Ballonsonde zur Bestimmung eines ösophagealen Drucks.

Ösophaguskatheter mit Ballonsonde (im Folgenden auch kurz als Ballonkathteter bezeichnet) zur Bestimmung eines ösophagealen Drucks werden insbesondere bei der maschinellen Beatmung eingesetzt, um den transpulmonalen Druck zu ermitteln.

Bei heute gängigen Formen der maschinellen Beatmung wird dem Patienten Atemgas mit Überdruck zugeführt. Deswegen ist bei der Beatmung der Atemwegsdruck bzw. alveoläre Druck zumindest während der Inspirationsphase größer als der Druck im die Lungenbläschen bzw. Alveolen umgebenden Pleuraspalt. Während der Expirationsphase erfolgt keine Druckbeaufschlagung des Atemwegs durch die Beatmungseinrichtung, mit der Folge, dass das Lungengewebe sich entspannt und der Atemwegsdruck bzw. der alveoläre Druck sinkt. Diese Art der Überdruckbeatmung kann unter bestimmten Umständen dazu führen, dass sich die Druckverhältnisse im Atemweg bzw. in den Alveolen am Ende der Expirationsphase derart ungünstig einstellen, dass es zu einem Kollaps von Teilen der Alveolen kommt. Dann muss der kollabierte Teil des Lungenvolumens im nachfolgenden Atemzyklus erst wieder von Neuem entfaltet werden. Die funktionelle Residualkapazität der Lunge wird stark beeinträchtigt, so dass die Sauerstoffsättigung abnimmt, und auch das Lungengewebe nimmt dauerhaft Schaden.

Um einen Kollaps von Alveolen am Ende der Expirationsphase zu verhindern, wird bei der maschinellen Überdruckbeatmung in der Regel ein sog. positiver endexpiratorischer Druck, in der Regel kurz als PEEP bezeichnet, eingestellt. Mit dieser Maßnahme kann in vielen Fällen eine Verbesserung der Sauerstoffsättigung erreicht werden.

Bei Beatmung mit PEEP beaufschlagt die Beatmungseinrichtung den Atemweg dauerhaft - also sowohl während der Inspirationsphase als auch während der Expirationsphase - mit einem vorbestimmten Überdruck, dem PEEP. Der PEEP liegt also auch nach dem Ende der Expirationsphase noch an.

Idealerweise sollte der PEEP genügend groß eingestellt werden, so dass während der Expirationsphase der alveoläre Druck nicht, oder jedenfalls nur so weit, unterhalb dem Druck im Pleuraspalt liegt, dass das alveoläre Gewebe unter der Wirkung des Drucks im Pleuraspalt nicht kollabiert. Mit anderen Worten: Der PEEP soll verhindern, dass der transpulmonale Druck - das ist die Druckdifferenz zwischen alveolärem Druck und Druck im Pleuraspalt - kleiner wird als Null bzw. einen unteren negativen Grenzwert unterschreitet, ab dem Teile der Alveolen zu kollabieren beginnen.

Andererseits kann sich ein zu hoher Wert des PEEP negativ auswirken, insbesondere während der Inspirationsphase. Denn das Lungengewebe kann bei sehr hohen Atemwegsdrücken während der Inspirationsphase überdehnt werden. Zahlreiche Studien deuten außerdem darauf hin, dass ein hoher Wert des PEEP den Rückfluss von venösem Blut zum Herzen behindern kann mit entsprechend negativen Auswirkungen auf das Herz-Kreislaufsystem.

An sich sollte der PEEP an den jeweils herrschenden transpulmonalen Druck angepasst sein. Der transpulmonale Druck bei einem beatmeten Patienten ist aber einer einfachen Bestimmung nicht zugänglich. Man behilft sich daher mit der Messung des Drucks in einer Ballonsonde, die mittels eines Ösophaguskatheters im Ösophagus eines zu beatmenden Patienten platziert wird. Bei geeigneter Positionierung und Konfiguration des Ballons lässt sich der im Ballon gemessene Druck näherungsweise zur Bestimmung des Drucks im Pleuraspalt heranziehen.

Die WO 2014/037175 A1 beschreibt die automatisierte Einstellung eines durch eine Beatmungseinrichtung vorgegebenen Drucks, insbesondere des positiven endexpiratorischen Drucks (PEEP) und des maximalen Atemwegsdrucks, auf Grundlage eines durch eine Messeinrichtung erfassten Drucks, der als Indikator für den transpulmonalen Druck, also die Druckdifferenz zwischen alveolärem Druck und Druck im Pleuraspalt, angesehen wird. Bei Bestimmung des transpulmonalen Drucks wird als Messgröße für den Druck im Pleuraspalt der Druck in einem in den Ösophagus eingeführten Ballonkatheter gemessen.

In der Praxis kann es vorkommen, dass die Beziehung zwischen dem Druck, der in einem in den Ösophagus eingeführten Ballonkatheter gemessen wird, und dem Druck im Pleuraspalt sich während der Beatmung verändert. Die Ursachen hierfür können mannigfaltig sein und sind in aller Regel nicht im Einzelnen ermittelbar.

Mojoli et al., Crit. Care (2016) 20:98, beschreiben eine Prozedur zur Kalibrierung eines zur Messung des ösophagealen Drucks vorgesehenen Ballonkatheters. Ziel der Kalibrierung ist es, eine optimale Befüllung des Ballonkatheters mit Luft zu erzielen, bei der der Ballonkatheter möglichst empfindlich auf Änderungen des auf den Ösophagus einwirkenden Drucks im Pleuraspalt reagiert und den Druck im Pleuraspalt möglichst gut wiedergibt. Die Kalibrierung erfolgt in vivo bei in den Ösophagus eingeführtem Ballonkatheter. Es werden nacheinander bei verschiedenen Mengen von in den Ballonkatheter eingefülltem Fluid (Luft) der vom Ballonkatheter gemessene Druck am Ende der Inspirationsphase und der vom Ballonkatheter gemessene Druck am Ende der Expirationsphase gemessen und die Differenz zwischen beiden Drücken bestimmt. Die optimale Ballonfüllung ergibt sich dann als das Maximum der Druckdifferenz in einem Messbereich, in dem sowohl der vom Ballonkatheter gemessene Druck am Ende der Inspirationsphase als auch der vom Ballonkatheter gemessene Druck am Ende der Expirationsphase quasi linear mit der Menge von in den Ballon gefülltem Fluid ansteigt. Um bei dieser in-vivo Kalibrierung reproduzierbare Ergebnisse erzielen zu können, wurde bei dieser Studie jede einzelne Ballonfüllung nach derselben Prozedur eingestellt, nämlich wie folgt: Ausgehend von einem leeren und mit der Umgebung in Druckausgleich gebrachten Ballonkatheter wurde zunächst der Ballonkatheter auf einen oberhalb des Messbereichs liegenden Wert überfüllt und sodann Fluid aus dem Ballon abgelassen, bis die gewünschte Ballonfüllung erreicht war. Dabei muss vor Aufnahme eines neuen Messpunktes immer darauf geachtet werden, dass ausreichend Zeit zum Ausgleich von Fluidströmungen, Drücken und/oder zum Ablauf von Relaxationsvorgängen im Kathetermaterial bzw. Ösophagusgewebe bleibt und somit instabile Verhältnisse vermieden werden. Die in Mojoli et al. beschriebene Messprozedur ist daher vergleichsweise aufwändig und empfindlich, so dass die Kalibrierung eines Ballonkathers eine erhebliche Zeitdauer von ungefähr 15 min oder sogar noch länger benötigt. In der Praxis führt dies dazu, dass lediglich eine optimale Befüllung für die Ballonsonde vor Beginn einer Beatmung bestimmt und voreingestellt werden kann. Diese optimale Befüllung wird dann während der Beatmung beibehalten und nicht mehr verändert.

Hatz et al., Respir. Care (2018) 63(2):177-186, empfehlen ebenfalls eine Kalibrierung der in den Ballonkatheter eingefüllten Fluidmenge, um eine maximale Empfindlichkeit des gemessenen Drucks auf Veränderungen des Drucks im Pleuraspalt zu erreichen und die Genauigkeit der Messung zu verbessern. Die dort vorgeschlagene in vivo Kalibrierung ist ähnlich der in Mojoli et al. vorgeschlagenen Kalibrierung, insbesondere ähnlich zeitaufwändig.

Die vorliegende Erfindung stellt ein Kalibriersystem bereit, mit dem eine Verbesserung der Genauigkeit von Druckmesswerten, die von einem in den Ösophagus eingeführten Ballonkatheter geliefert werden, erzielt wird. Insbesondere erlaubt das erfindungsgemäße Kalibriersystem eine genauere Wiedergabe des im Pleuraspalt herrschenden Drucks durch den Ballonkatheter und schnellere Reaktion der Messwerte auf sich verändernde Umgebungsbedingungen während andauernder Beatmung. Damit wird eine Kalibrierung des Ballonkatheters während der Beatmung möglich, ohne dass die Beatmung dazu unterbrochen werden muss. Dies gilt selbst dann, wenn die Beatmung mittels vollautomatischer Beatmungsmodi erfolgt, beispielsweise bei Beatmung mittels geschlossener Regelkreise, wie bei der von der Anmelderin entwickelten Adaptive Support Ventilation (ASV-Beatmung).

Erfindungsgemäß wird ein Kalibriersystem vorgeschlagen, welches zur automatisierten Einstellung einer betriebsgemäßen Befüllung eines in den Ösophagus einführbaren Ösophaguskatheters mit Ballonsonde zur Bestimmung eines ösophagealen Drucks, insbesondere für eine Beatmungsvorrichtung, ausgebildet ist. Das Kalibriersystem umfasst folgende Komponenten:
- eine Anordnung zum Befüllen der Ballonsonde mit einem Messfluid nach Platzieren der Ballonsonde im Ösophagus,
- einen Drucksensor zum Erfassen eines in der Ballonsonde herrschenden ösophagealen Drucks,
- eine Kalibriersteuerung die derart ausgebildet ist, dass sie die Menge an Messfluid in der Ballonsonde schrittweise verändert, wobei die Kalibriersteuerung bei jeder der auf diese Weise schrittweise als Messpunkte eingestellten Mengen an Messfluid in der Ballonsonde einen von dem Drucksensor erfassten ösophagealen
Drucks aufnimmt und der jeweils eingestellten Menge an Messfluid in der Ballonsonde zuordnet.

Die Kalibriersteuerung ist derart ausgebildet, dass sie zum Anfahren der jeweiligen Messpunkte die Menge an Messfluid ausgehend von einem Startwert bis zum Erreichen eines Endwertes in mindestens zwei Schritten in monotoner Weise verändert.

Das Messfluid ist insbesondere Luft.

Das Kalibriersystem kann insbesondere auch eine Anordnung zum Entnehmen von Messfluid aus der Ballonsonde (Fluidablasseinrichtung) nach Platzieren der Ballonsonde im Ösophagus umfassen. Sowohl die Anordnung zum Befüllen der Ballonsonde mit einem Messfluid nach Platzieren der Ballonsonde im Ösophagus als auch die Anordnung zum Entnehmen von Messfluid aus der Ballonsonde nach Platzieren der Ballonsonde im Ösophagus können eine oder mehrere Ventile aufweisen, das/die in einer mit der Ballonsonde in Fluidverbindung stehenden Förderleitung für Messfluid angeordnet ist/sind. Soweit das Messfluid in der Ballonsonde unter einem Überdruck steht, kann die Fluidablasseinrichtung einfach durch Ansteuerung von Ventilen in einer mit der Ballonsonde in Fluidverbindung stehenden Fluidleitung realisiert sein.

Die Anordnung zum Befüllen der Ballonsonde mit einem Messfluid nach Platzieren der Ballonsonde im Ösophagus kann eine Pumpvorrichtung umfassen, mittels derer Messfluid in die Ballonsonde gepumpt werden kann. In diesem Fall kann die Pumpvorrichtung auch zum Entnehmen (Abpumpen) von Messfluid aus der Ballonsonde ausgebildet sein. Die Fluidablasseinrichtung kann die Pumpvorrichtung aufweisen.

In weiterer Ausgestaltung kann die Anordnung zum Befüllen der Ballonsonde mit einem Messfluid und/oder die Anordnung zum Entnehmen von Messfluid aus der Ballonsonde einen Flusssensor aufweisen, insbesondere einen Massenflusssensor, welcher zur Bestimmung einer Menge an in die Ballonsonde eingeleitetem und/oder einer Menge an aus der Ballonsonde entnommenen Messfluid ausgebildet ist. Beispielsweise kann durch Integrieren eines von dem Flusssensor gemessenen Flusses über einen Zeitraum zwischen einem Startzeitpunkt und einem Endzeitpunkt die jeweils in die Ballonsonde eingeleitete bzw. aus der Ballonsonde entnommene Menge an Messfluid bestimmt werden. Wenn ein Flusssensor eingesetzt wird, der den Fluss an Messfluid anhand eines Differenzdrucks bestimmt, kann der Flusssensor auch zur Erfassung des in der Ballonsonde herrschenden ösophagealen Drucks eingesetzt werden. Ein separater Drucksensor ist ebenfalls möglich.

Die Kalibriersteuerung kann als eigenständiges Bauteil "in Hardware" realisiert werden. Alternativ kann die Kalibriersteuerung auch als Computerprogrammprodukt, d.h. durch ein entsprechendes Softwareprogramm realisiert werden, das auf einem Prozessor, insbesondere einem Mikroprozessor oder Mikrocontroller, ausgeführt wird. In diesem Fall kann die Software auf einem geeigneten lokalen oder über ein Netzwerk abrufbaren Speichermedium vorgehalten werden. Die Software enthält als Computerprogramm codierte Anweisungen, die dann, wenn die Software in einen Arbeitsspeicher des Prozessors geladen und in Maschinensprache übersetzt wird, den Prozessor dazu veranlasst, die hierin näher beschriebenen Prozeduren auszuführen. Mischformen zwischen Realisierung in Hardware und Realisierung in Software sind selbstverständlich ebenso denkbar. Der Mikroprozessor oder Mikrocomputer kann zu einer Steuerung des Kalibriersystems gehören.

Mit dem Begriff "in monotoner Weise" soll zum Ausdruck gebracht werden, dass die Menge an Messfluid in der Ballonsonde sich während eines Messzyklus immer in derselben Richtung verändert. Das heißt, die Menge an Messfluid nimmt während eines Messzyklus zwischen Startwert und Endwert zum Erreichen jedes weiteren Messpunktes entweder immer weiter ab oder immer weiter zu. Dabei definiert das Anfahren von Messpunkten zwischen dem Startwert und dem Endwert einen Messzyklus. Startwert und Endwert eines Messzyklus können durch vorbestimmte Mengen an in die Ballonsonde geleitetem bzw. aus der Ballonsonde geleitetem Messfluid definiert sein. Alternativ ist auch denkbar, Startwert und Endwert durch vorbestimmte Werte des in der Ballonsonde erfassten Drucks festzulegen. Gleiches gilt auch für die einzelnen Messpunkte, die zwischen Startwert und Endwert eines Messzyklus angefahren werden. Es hat sich herausgestellt, dass es einfacher und schneller ist, jedenfalls beim Anfahren aufeinander folgender Messpunkte zwischen dem Startwert und dem Endwert (wobei Start - und Endwert ggf. nicht einbegriffen sind) immer eine vorbestimmte Menge an Messfluid aus der Ballonsonde abzuleiten bzw. in die Ballonsonde zu einzuleiten.

Das erfindungsgemäß vorgeschlagene monotone Durchschreiten des Messbereichs zwischen Startwert und Endwert innerhalb eines Messzyklus beschleunigt die Kalibrierung enorm. Dies liegt insbesondere daran, dass aufeinander folgende Messpunkte innerhalb eines Messzyklus ausgehend vom Startwert bis mindestens zum Endwert unmittelbar nacheinander angefahren werden können. Man benötigt insbesondere keine Zwischenschritte zum Evakuieren der Ballonsonde und/oder zum Einstellen von jeweils genau reproduzierbaren Anfangsbedingungen für die einzelnen Messpunkte während einer Kalibration mehr. Zudem müssen keine Druckausgleichsvorgänge oder Relaxationsvorgänge mehr abgewartet werden. Es hat sich überraschender Weise gezeigt, dass die Kalibrierung durch das Weglassen einer für jeden Messpunkt gleichen Prozedur mit denselben Anfangsbedingungen für jeden Messpunkt keinen - jedenfalls nicht inakzeptabel großen - negativen Einflüssen unterliegt. Insbesondere scheinen sich Hystereseeffekte - soweit vorhanden - auf alle Messpunkte innerhalb eines Messzyklus in etwa gleich auswirken und deshalb die Kalibrierung nicht zu stören.

Besondere Ausgestaltungen des hier vorgeschlagenen Kalibriersystems können eines oder mehrere der nachfolgend dargelegten optionalen Merkmale aufweisen. Soweit Merkmale ausschließlich alternative Ausgestaltungen betreffen, ist dies im Folgenden ausdrücklich vermerkt. Es versteht sich daher, dass die nachfolgenden Merkmale in beliebiger Weise mit den jeweils vorangehend oder nachfolgend dargelegten Merkmalen kombinierbar sind, soweit nicht ausdrücklich ausgeschlossen.

Wie bereits angesprochen, kann das Kalibriersystem ferner eine Fluidablasseinrichtung umfassen, die zum Ablassen von Messfluid aus der Ballonsonde ausgebildet ist. Dabei steuert die Kalibriersteuerung die Fluidablasseinrichtung zum Anfahren der jeweiligen Messpunkte derart an, dass die Menge an Messfluid in der Ballonsonde ausgehend von dem Startwert bis zum Erreichen des Endwertes in mindestens zwei Schritten in monotoner Weise abnimmt. Das Ansteuern kann beispielsweise durch zeitweiliges Öffnen von Ventilen in einer mit der Ballonsonde in Fluidverbindung stehenden Förderleitung erfolgen. Auch aktives Pumpen mittels einer entsprechenden Pumpeinrichtung ist denkbar.

Die Kalibriersteuerung kann ferner derart ausgebildet sein, dass sie die Anordnung zum Befüllen der Ballonsonde mit einem Messfluid wenigstens in einem ersten Messzyklus zum Befüllen der Ballonsonde mit einer Menge an Messfluid derart ansteuert, dass die Menge an in die Ballonsonde eingeleitetem Messfluid größer ist als eine obere Grenze des dem Messzyklus zugeordneten Messbereichs zwischen Startwert und Endwert. In diesem Fall wird der Startwert, der den Beginn des Messzyklus definiert, erst nach Ablassen einer ersten Menge an Messfluid aus der Ballonsonde angefahren. Die Kalibriersteuerung kann die Anordnung zum Befüllen der Ballonsonde derart ansteuern, dass diese die Ballonsonde zunächst mit einer größeren Menge an Messfluid befüllt als die dem Startwert entsprechende Menge an Messfluid. Die Kalibriersteuerung kann dann die Fluidablasseinrichtung derart ansteuern, dass diese Messfluid aus der Ballonsonde ablässt, bis sich in der Ballonsonde die dem Startwert entsprechende Menge an Messfluid befindet.

Diese Maßnahmen führen zu einem gewissen Überdehnen der Ballonsonde vor Beginn des anstehenden Messzyklus. Damit ist sichergestellt, dass der Messzyklus tatsächlich den gesamten als Messbereich nutzbaren Bereich abdeckt, in dem sich der von der Ballonsonde gemessene Druck ungefähr linear mit Menge an Messfluid in der Ballonsonde ändert. Bei der vorgeschlagenen Vorgehensweise mit Überdehnen der Ballonsonde vor Anfahren des Startwerts nimmt also während des Messzyklus zumindest in einem mittleren Bereich der in der Ballonsonde gemessene Druck ungefähr linear mit Menge an Messfluid in der Ballonsonde ab. Diese lineare Abnahme legt den grundsätzlich als Messbereich für den Ösophaguskatheter nutzbaren Bereich zwischen einer oberen Füllmenge an Messfluid in der Ballonsonde und einer unteren Füllmenge an Messfluid in der Ballonsonde fest.

Da die Messung in vivo, also bei im Ösophagus eines Patienten platzierter Ballonsonde erfolgt, wird im als Messbereich herangezogenen linearen Teil die Steigung der ösophagealer Druck/Messfluidmenge-Kurve mehr von der Dehnbarkeit des Ösophagus und weniger von der Dehnbarkeit der Ballonsonde bestimmt. Der Ösophagus dehnt sich im linearen Teil mit abnehmender Messfluidmenge immer weniger bzw. verringert seinen Querschnitt mit abnehmender Messfluidmenge bei in etwa gleichbleibender Dehnbarkeit.

Im Bereich oberhalb des linearen Teils des Messbereichs ist - jedenfalls bei ausreichend großem Durchmesser der Ballonsonde - die maximale Dehnbarkeit des Ösophagus erreicht. Die Ballonsonde kann sich dann bei weiterer Erhöhung der eingefüllten Fluidmenge kaum mehr weiter dehnen. Daher steigt der Druck im Ösophagusballon mit zunehmender Messfluidmenge steiler an als im linearen Teil des Messbereichs. In der Regel wird, jedenfalls in einem ersten Messzyklus, der Startwert für die Messfluidmenge so gewählt sein, dass der Startwert für den gemessenen ösophagealen Druck in dem Bereich oberhalb des linearen Teils des Messbereichs liegt.

In weiterer Ausgestaltung kann die Kalibriersteuerung dazu ausgebildet sein, mindestens zwei Messzyklen nacheinander auszuführen. Dabei kann der Messbereich der mindestens zwei aufeinanderfolgenden Messzyklen verschieden sein. Insbesondere kann ein vorangehender Messzyklus den Messbereich für einen nachfolgenden Messzyklus festlegen.

Beispielsweise kann im vorangehenden Messzyklus ein erster Messbereich zwischen einem ersten Startwert und einem ersten Endwert durchfahren werden, um in etwa den linearen Bereich zu finden, in dem die Dehnbarkeit des Ösophagus in etwa gleichbleibt. Danach kann im nachfolgenden Messzyklus eine feinere Abstufung der Messpunkte zwischen einem zweiten Startwert und einem zweiten Endwert, die beide im linearen Bereich liegen, erfolgen.

Die Kalibriersteuerung kann ferner derart ausgebildet sein, dass sie den Abstand zwischen aufeinanderfolgenden Messpunkten für den vorangehenden Messzyklus und für den nachfolgenden Messzyklus unterschiedlich einstellt. Dabei soll sich der Begriff "Abstand zwischen Messpunkten" auf die Differenz zwischen den zugehörigen Mengen an Messfluid in der Ballonsonde beziehen.

Beispielsweise kann in einem vorangehenden Messzyklus ein relativ weiter Abstand zwischen aufeinanderfolgenden Messpunkten eingestellt sein, um den Bereich zu identifizieren, in dem der im Ösophaguskatheter ermittelte ösophageale Druck sich in etwa linear mit der Menge an Messfluid in der Ballonsonde verändert. In einem nachfolgenden Messzyklus kann dann dieser lineare Bereich zwischen der zugehörigen maximalen Menge an Messfluid und minimalen Menge an Messfluid mit geringerer Schrittweite durchfahren werden, um die optimale Befüllungsmenge an Messfluid für den Ösophaguskatheter zu finden.

Ferner kann die Kalibriersteuerung dazu ausgebildet sein, die Schrittweite zwischen aufeinander folgenden Messpunkten innerhalb des Messbereichs in einem Messzyklus in adaptiver Weise zu bestimmen. Eine solche adaptive Bestimmung der Schrittweite kann beispielsweise anhand eines Gradientenverfahrens wie dem Newton-Algorithmus erfolgen.

Bei mehreren aufeinanderfolgenden Messzyklen kann es vorteilhaft sein, die bestimmte "Überdehnung" der Ballonsonde am Anfang, also das anfängliche Einleiten einer größeren Menge an Messfluid als es dem Startwert des jeweiligen Messzyklus entspricht, vor jedem Messzyklus durchzuführen. Dann kann nämlich die Kalibriersteuerung derart ausgebildet sein, dass zwischen einem vorangehenden Messzyklus und einem nachfolgenden Messzyklus keine vollständige Entleerung des Messfluids aus der Ballonsonde stattfindet. Es findet in solchen Ausführungsformen insbesondere kein vollständiges Evakuieren des von der Ballonson-de umschlossenen Hohlraums bzw. Volumens statt, auch nicht zwischen aufeinander folgenden Messzyklen. Man macht sich vielmehr zu Nutze, dass es auf die genaue Menge an vor Beginn des jeweiligen Messzyklus in der Ballonsonde befindlichen Messfluid nicht ankommt, wenn man ausgehend von einem beliebigen Startwert am Anfang eine größere Menge an Messfluid in die Ballonsonde einleitet, als für den Startwert benötigt wird, weil die so bewirkte Überdehnung viel mehr von der Menge an in die Ballonsonde eingeleitetem Messfluid abhängt als vom Anfangszustand, von dem aus die Befüllung gestartet ist. Zudem kommt es häufig auch auf den genauen Grad der Überdehnung der Ballonsonde gar nicht an, weil man beim zum Erreichen des Startwerts für den nachfolgenden Messzyklus erforderlichen Entnehmen von Messfluid aus der Ballonsonde den für den jeweiligen Messzyklus gewünschten Startwert anhand des erfassten ösophagealen Drucks erkennen kann.

Zur Verbesserung der Genauigkeit kann es allerdings auch vorgesehen sein, vor Beginn eines Messzyklus, oder wenigstens vor Beginn eines ersten Messzyklus bei mehreren aufeinander folgenden Messzyklen, die Ballonsonde auf einen vorbestimmten "Nullzustand" einzustellen, beispielsweise durch Abpumpen oder andersartiges Ableiten von Messfluid aus der Ballonsonde, bis ein vorbestimmter Unterdruck (z.B. - 20hPa gegenüber Umgebungsdruck) erreicht ist. Auf diese Weise kann sichergestellt werden, dass die Kalibrierprozedur von einem vorbestimmten Anfangszustand der Ballonsonde aus startet, in dem beispielsweise sicher ist, dass die Ballonsonde eine vollständig zusammengefaltete Konfiguration aufweist. Ausgehend von diesem Nullzustand kann dann Messfluid in die Ballonsonde eingeleitet bzw. gepumpt werden, bis eine Überfüllung der Ballonsonde auf einen vorbestimmten Druck, der oberhalb des Startwerts für den ersten Messzyklus liegt, erfolgt ist. Von diesem Zustand aus kann dann die Kalibrierprozedur in der vorangehend beschriebenen Weise durch aufeinander folgendes Anfahren von mehreren Messpunkten in monotoner Weise zwischen einem Startwert und einem Endwert erfolgen.

In weiteren Ausführungsformen kann vorgesehen sein, dass die Kalibriersteuerung derart ausgebildet ist, dass sie für jeden Messpunkt, d.h. für jede eingestellte Menge an Messfluid in der Ballonsonde zwischen dem Startwert und dem Endwert eines Messzyklus (falls gewünscht, einschließlich des Startwerts und des Endwerts), einen Messwert für den ösophagealen Druck am Ende einer Inspirationsphase und einen Messwert für den ösophagealen Druck am Ende einer Expirationsphase ermittelt und dann die Differenz zwischen dem ösophagealen Druck am Ende der Inspirationsphase und dem ösophagealen Druck am Ende der Expirationsphase bestimmt.

Dabei kann die Kalibriersteuerung derart ausgebildet sein, dass sie für einen zwischen dem Startwert für die Menge an Messfluid in der Ballonsonde und dem Endwert für die Menge an Messfluid in der Ballonsonde liegenden Bereich (falls gewünscht, einschließlich des Startwerts und des Endwerts) einen Maximalwert für die Differenz zwischen dem ösophagealen Druck am Ende der Inspirationsphase und dem ösophagealen Druck am Ende der Expirationsphase bestimmt (Maximaldruck). Die dem Maximaldruck entsprechende Menge an Messfluid in der Ballonsonde entspricht dann der optimalen Befüllung für die Ballonsonde, d.h. der Menge an Messfluid in der Ballonsonde, mit der die Beatmung durchgeführt werden sollte.

Ferner kann die Kalibriersteuerung derart ausgebildet sein, dass sie für einen jeweiligen Messpunkt zwischen dem Startwert und dem Endwert den Messwert für den ösophagealen Druck am Ende einer Inspirationsphase und den Messwert für den ösophagealen Druck am Ende einer Expirationsphase bei weiterlaufender Beatmung ermittelt.

Ein laufender Beatmungszyklus bzw. Atemzyklus braucht somit zur Ermittlung eines Messwerts für den ösophagealen Druck nicht unterbrochen zu werden. Es ist insbesondere zur Ermittlung dieser Messwerte keine Einstellung einer Totzeit erforderlich, in der die Atemwege verschlossen werden, so dass der Fluss an Beatmungsgas bzw. Atemgas zum Erliegen kommt. Die Beatmung eines Patienten kann somit unverändert weiterlaufen, während eine Kalibrierung bzw. Neukalibrierung des Ösophaguskatheters durchgeführt wird. Dies ist ein großer Vorteil, nicht nur, weil jede Unterbrechung oder Störung der Beatmung aus Patientensicht zu vermeiden ist, sondern auch weil die Kalibrierung des Ösophaguskatheters auf diese Weise unter möglichst lebensnahen Bedingungen erfolgen kann.

Ferner kann die Kalibriersteuerung derart ausgebildet sein, dass sie die für die jeweiligen Messpunkte ermittelten Differenzen zwischen dem ösophagealen Druck am Ende der Inspirationsphase und dem ösophagealen Druck am Ende der Expirationsphase miteinander vergleicht, und dann, wenn über eine Mehrzahl von aufeinander folgenden Messpunkten die für einen jeweiligen Messpunkt ermittelte Druckdifferenz innerhalb einer vorgegebenen Schwankungsbreite bezüglich eines Maximalwerts der Druckdifferenz liegt, die einer optimalen Befüllung der Ballonsonde entsprechende Menge an Messfluid als eine Menge an Messfluid bestimmt, die einen vorgegebenen Abstand zur kleinsten Menge an Messfluid in der Ballonsonde (unterer Rand) und/oder zur größten Menge an Messfluid in der Ballonsonde (oberer Rand), bei der die erfasste Druckdifferenz noch in die Schwankungsbreite fällt, hat.

Beispielsweise kann vorgegeben sein, dass die der optimalen Befüllung der Ballonsonde entsprechende Menge an Messfluid näher am unteren Rand eines solchen Bereichs mit in etwa gleichen Druckdifferenzen in der Nähe des Maximalwertes liegt als am oberen Rand. Die der optimalen Befüllung der Ballonsonde entsprechende Menge an Messfluid in der Ballonsonde ("optimale Befüllung") kann beispielsweise um 1/3 des Abstands zwischen der dem oberen Rand des Bereichs mit in etwa gleicher Druckdifferenz entsprechenden Menge an Messfluid in der Ballonsonde und der dem unteren Rand des Bereichs in etwa gleicher Druckdifferenz entsprechenden Menge an Messfluid in der Ballonsonde größer sein als die dem unteren Rand des Bereichs mit in etwa gleicher Druckdifferenz entsprechende Menge an Messfluid.

Insbesondere kann die Kalibriersteuerung derart ausgebildet sein, dass sie für jeden Messpunkt zwischen dem Startwert und dem Endwert (ggf. einschließlich Startwert und Endwert) eine Mehrzahl von Messwerten, insbesondere eine Mehrzahl von Messwertpaaren für den ösophagealen Druck am Ende einer Inspirationsphase und für den ösophagealen Druck am Ende einer Expirationsphase ermittelt. Dies wird in der Regel in aufeinander folgenden Beatmungszyklen geschehen. Dann kann die Kalibriersteuerung anhand der Mehrzahl von Messwerten für jeden Messpunkt einen Durchschnitt und eine statistische Streuung für den Messwert oder eine daraus abgeleitete Größe, insbesondere die Differenz zwischen dem ösophagealen Druck am Ende der Inspirationsphase und dem ösophagealen Druck am Ende der Expirationsphase (Druckdifferenz), bestimmen und die Anzahl von Messungen je Messpunkt so festlegen, dass der erhaltene Durchschnitt als statistisch signifikant angesehen werden kann.

Generell gilt: Je stärker die statistische Schwankung der erhaltenen Messwerte ist, desto mehr Atemzyklen werden für die Ermittlung des einem jeweiligen Messpunkt zugeordneten Messwerts für die Druckdifferenz zugrunde gelegt. Hier können beispielsweise kardiogene Oszillationen oder Bewegungen eine Rolle spielen. Solche Effekte können durch Erhöhung der Anzahl von Atemzyklen pro Messung besser unterschieden werden. Als Durchschnitt kann insbesondere ein arithmetisches Mittel zugrunde gelegt werden. Aber auch andere Arten der Durchschnittsbildung sind denkbar, beispielsweise ein geometrischer Durchschnitt.

Die statistische Streuung kann insbesondere durch eine Gaussche Standardabweichung repräsentiert sein. Dann wäre es denkbar, als statistisch signifikant ein 2-Sigma Niveau festzulegen, also eine Gaussche Standardabweichung von 95% oder besser also signifikant zu werten. Eine weitere Variante könnte darin bestehen, zur Ermittlung der Streuung adaptiv vorzugehen, beispielsweise indem für einen jeweiligen Messpunkt die Änderung des Mittelwertes nach jedem neuen Messwert als Maß ist für Signifikanz herangezogen wird. Je kleiner die Änderung des Mittelwerts nach einem jeweiligen Messwert ist, desto höher ist die Signifikanz. Es können für einen jeweiligen Messpunkt solange Messungen wiederholt werden, bis die Änderung des Mittelwerts nach der letzten Messung kleiner geworden ist als ein vorbestimmter Schwellenwert. Danach kann zum nächsten Messpunkt übergegangen werden, etwa durch Entnehmen einer vorbestimmten Menge an Messfluid aus der Ballonsonde.

In weiteren Ausführungsformen kann die Kalibriersteuerung derart ausgebildet sein, dass sie für jeden Messpunkt zwischen dem Startwert und dem Endwert überwacht, ob die jeweilige Messung (bei mehrmaliger Messung einer Druckdifferenz pro Messpunkt bei jeder dieser Messungen) durch äußere Umstände beeinträchtigt ist, und die jeweilige Messung verwirft, wenn solche äußeren Umstände festgestellt werden. Äußere Umstände können beispielsweise Schluckbemühungen eines Patienten sein. Dabei kann vorgesehen sein, dass nicht die gesamte Kalibrierung verworfen oder abgebrochen wird, sondern lediglich der durch äußere Umstände beeinträchtige Messwert verworfen wird. Insbesondere kann ein neuer Messwert für den jeweiligen Messpunkt unmittelbar nachfolgend ermittelt werden. Danach kann die Kalibrierungsprozedur normal weiterlaufen. Es werden also artifizielle Messwerte "in Echtzeit" eliminiert, ohne dass die Kalibrierungsprozedur abgebrochen oder für längere Zeit unterbrochen werden muss. Ein erneutes Starten der gesamten Kalibrierung nach Erkennen von äußeren Umständen würde deutlich länger dauern und könnte, beispielsweise im Fall von Schluckbemühungen des Patienten während einer oder mehrerer Messungen zudem dazu führen, dass erneut Schluckbemühungen beim Patienten ausgelöst werden. Dies ist gegenüber bekannten Ansätzen ein enormer Fortschritt, denn bei den bekannten Ansätzen ist entweder die Entscheidung einer Bedienperson erforderlich, was jede Automatisierung unmöglich macht, oder es müssen automatisiert ablaufende Kalibrierungen ohne Berücksichtigung äußerer Umstände durchgezogen werden. Erst hinterher wird eine Kalibrierung komplett verworfen, wenn durch Analyse der Daten im Raum steht, dass wenigstens einer oder einige der Messwerte, die in die fragliche Kalibrierung eigeflossen sind, möglicherweise von äußeren Einflüssen wie Schluckbemühungen o.ä. beeinträchtigt sind. Es lässt sich also nicht mit Sicherheit und bereits vor Beginn einer Kalibrierung sagen, ob diese Kalibrierung gültig ist oder möglicherweise verworfen werden muss.

Ungewöhnliche äußere Umstände wie Schluckbemühungen des Patienten können beispielsweise durch Überwachung des zeitlichen Verlaufs des ösophagealen Drucks erkannt werden, insbesondere wenn am Ende der maschinell vorgegebenen Inspirationsphase und/oder am Ende der maschinell vorgegebenen Expirationsphase Veränderungen des Drucksignals auftreten.

Die Kalibriersteuerung kann derart ausgebildet sein, dass sie bei Auftreten solcher Störungen die Kalibrierung unterbricht und erst wieder fortsetzt, wenn anhand des erfassten ösophagealen Drucks und/oder Drucks im Atemweg feststellbar ist, dass keine weiteren Störungen mehr auftreten.

Ferner kann die Kalibriersteuerung dazu ausgebildet sein, anhand der während der Kalibrierprozedur ermittelten Daten einen Qualitätsindex zu berechnen. Der Qualitätsindex kann eine gewichtete Zusammenfassung der Einflüsse verschiedener Faktoren darstellen. In solchen Ausführungsformen kann anhand des Qualitätsindex entschieden werden, ob Änderungen von Beatmungsparametern auf Grundlage von Daten des ösophagealen Drucks zugelassen werden oder nicht. Für den Qualitätsindex relevante Kriterien können die statistische Streuung der Messdaten oder ein ungewöhnlicher Verlauf der ösophagealer Druck/Messfluidmenge-Kurve sein. Als ein Maß für den Qualitätsindex kann die Streuung der Messwerte, beispielsweise ausgedrückt als Gaussche Standardabweichung herangezogen werden. Darüber hinaus können bestimmte Regeln festgelegt werden, die in den Qualitätsindex einfließen. Beispiele solcher Regeln können sein: Wenn während einer Kalibierprozedur ein Schlucken des Patienten erkannt wird, wird der Qualitätsindex reduziert. Wenn das Maximum der Differenz zwischen dem erfassten ösophagealen Druck am Ende der Inspiration und dem erfassten ösophagealen Druck am Ende der Expiration bei einem Messpunkt außerhalb, insbesondere oberhalb des linearen Bereichs der Kurve für die Beziehung zwischen ösophagealem Druck und Menge an Messfluid in der Ballonsonde liegt, wird der Qualitätsindex reduziert. Wenn der Basisdruck, d.h. der ösophageale Druck am Ende der Expirationsphase im Laufe aufeinander folgender Messungen nicht gleich bleibt oder sich stetig ändert, sondern Sprünge macht, wird der Qualitätsindex reduziert. Wenn ein bei einem höheren Wert an Fluidmenge in der Ballonsonde erfasster ösophagealer Druck kleiner ist als der Wert bei der niedrigeren Fluidmenge in der Ballonsonde, wird der Qualitätsindex reduziert.

Ferner kann die Kalibriersteuerung derart ausgebildet sein, dass der ösophageale Druck einen vorgegebenen Maximaldruck nicht überschreiten darf. Beispielsweise kann vorgesehen sein, dass der ösophageale Druck maximal das Doppelte des maximalen Atemwegsdrucks bei der Beatmung betragen darf. Bei Erreichen dieses Drucks wird kein weiteres Fluid in die Ballonsonde geleitet bzw. Fluid aus der Ballonsonde abgelassen. Auf diese Weise soll übermäßige Überdehnung am Gewebe des Ösophagus vermieden werden. Auch eine übermäßige Überdehnung der Ballonsonde, die zu deren Beschädigung führen könnte, kann so vermieden werden.

Schließlich kann vorgesehen sein, dass die Kalibriersteuerung eine bereits oben angesprochene Ablasseinrichtung, durch welche Messfluid aus der Ballonsonde ableitbar ist, beim Anfahren der jeweiligen Messpunkte derart ansteuert, dass die Menge an Messfluid in der Ballonsonde ausgehend von dem Startwert schrittweise immer weiter verringert wird, bis bei Erreichen oder Unterschreiten eines vorgegebenen minimalen endexpiratorischen ösophagealen Drucks, beispielsweise - 5 hPa, der Endwert erreicht ist.

Die vorliegende Erfindung betrifft ferner ein Verfahren zur automatisierten Kalibrierung einer betriebsgemäßen Befüllung eines in den Ösophagus einführbaren Ösophaguskatheters mit Ballonsonde zur Bestimmung eines ösophagealen Drucks, insbesondere für eine Beatmungsvorrichtung.

Dieses Verfahren umfasst folgende Schritte:
- Befüllen der Ballonsonde mit einem Messfluid nach Platzieren der Ballonsonde im Ösophagus, und
- Erfassen eines in der Ballonsonde herrschenden ösophagealen Drucks, sowie
- Schrittweises Verändern einer Menge an Messfluid in der Ballonsonde, wobei bei jeder der auf diese Weise schrittweise als Messpunkte eingestellten Mengen an Messfluid in der Ballonsonde der ösophageale Druck erfasst und der jeweils eingestellten Menge an Messfluid in der Ballonsonde zugeordnet wird.

Bei dem erfindungsgemäßen Verfahren wird zum Anfahren der jeweiligen Messpunkte die Menge an Messfluid ausgehend von einem Startwert bis zum Erreichen eines Endwertes in mindestens zwei Schritten in monotoner Weise verändert.

Das erfindungsgemäße Verfahren kann in weiteren Ausführungsformen wenigstens einen, insbesondere mehrere, der vorangehend mit Bezug auf eine Ausbildung eines Kalibriersystems implizit genannten weiteren Verfahrensschritte aufweisen. Zur Vermeidung von Wiederholungen wird ausdrücklich auf die ausführliche Darlegung dieser Verfahrensschritte in Bezug auf funktionale Merkmale des Kalibriersystems, insbesondere der Kalibriersteuerung verwiesen.

Weiterhin betrifft die vorliegende Erfindung ein Computerprogrammprodukt, welches Programmanweisungen enthält, bei deren Ausführung auf einer Datenverarbeitungsanlage, insbesondere auf einem Mikroprozessor oder einem Mikrocontroller zur Steuerung eines Ösophaguskatheters mit Ballonsonde, ein erfindungsgemäßes Kalibrierverfahren ausgeführt wird bzw. ein erfindungsgemäßes Kalibriersystem realisiert wird.

Weitere Ausführungsformen der vorliegenden Erfindung werden nachfolgend unter Bezugnahme auf die Figuren näher erläutert.
Fig. 1 zeigt in stark schematisierter Darstellung die wesentlichen Elemente einer Beatmungsvorrichtung samt intubierter Trachea und Thorax eines beatmeten Patienten;
Fig. 2 zeigt den zeitlichen Verlauf des Atemwegeingangsdrucks Paw (oben), ösophagealen Drucks Peso (Mitte) und der Differenz Paw - Peso aus beiden Drücken während mehrerer aufeinander folgender Atemzyklen bei maschineller Beatmung einschließlich Okklusionsmanövern;
Fig. 3 zeigt anhand eines Flussdiagramms den Ablauf einer Kalibrierung eines Ösophaguskatheters in vivo gemäß einer Ausführungsform der vorliegenden Erfindung.
Fig. 4 zeigt schematisch die während einer Kalibrierung gemäß einer Ausführungsform der vorliegenden Erfindung eingestellte Menge an Messfluid in der Ballonsonde des Ösophaguskatheters sowie den erfassten Druck in der Ballonsonde des Ösophaguskatheters; und
Fig. 5 zeigt in den Teilbildern a) und b) schematisch für jede eingestellte Menge an Messfluid in der Ballonsonde während eines Messzyklus den jeweils erfassten Druck in der Ballonsonde am Ende der Inspirationsphase, den jeweils erfassten Druck in der Ballonsonde am Ende der Expirationsphase (Teilbild a)), sowie den sich daraus jeweils ergebenden Differenzdruck zwischen Druck in der Ballonsonde
am Ende der Inspirationsphase und Druck in der Ballonsonde am Ende der Expirationsphase (Teilbild b)).

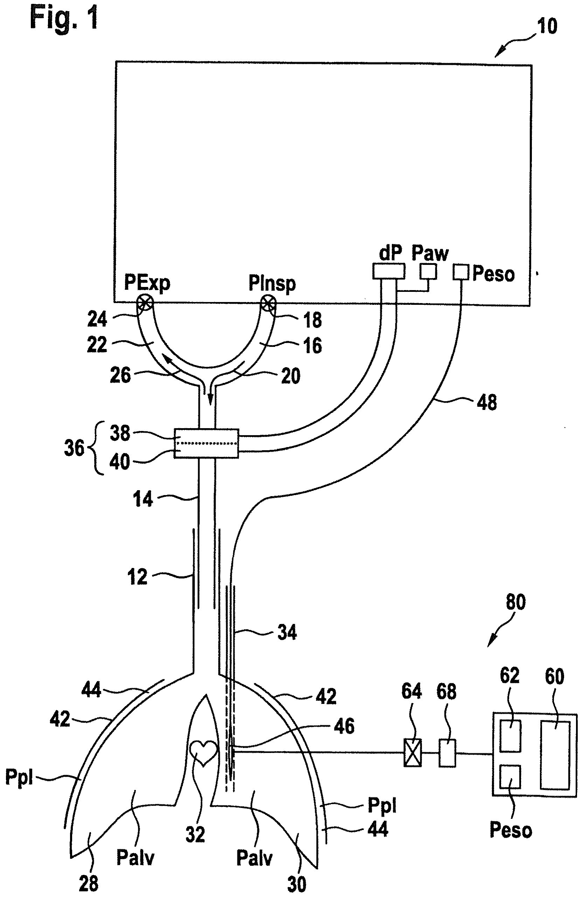

Fig. 1 zeigt in stark schematisierter Darstellung und in Form eines Blockdiagramms die wesentlichen Elemente einer Beatmungsvorrichtung 10. Die Beatmungsvorrichtung 10 ist in Fig. 1 in einem Zustand mit intubierter Luftröhre (Trachea) 12 eines beatmeten Patienten gezeigt. Neben der Trachea 12 sind in Fig. 1 noch die Lungenlappen 28, 30, das Herz 32, die Speiseröhre (Ösophagus) 34 und die Thoraxwand 42 des Patienten sehr schematisch angedeutet. Der Tubus 14 der Beatmungsvorrichtung 10 ist, in der Regel über die nicht gezeichnete Mundöffnung des Patienten, ein Stück weit in die Trachea 12 eingeschoben, um den Atemweg mit Atemgas zu beaufschlagen. Ausgeatmete Luft wird ebenfalls über den Tubus 14 abgeleitet, der sich an seinem stromaufwärtigen Ende in ein erstes Ende 16 und ein zweites Ende 22 verzweigt. Das erste Ende 16 ist über ein Atemwegeingangsventil 18 mit einem Atemwegeingangsanschluss der Beatmungsvorrichtung 10 zur Beaufschlagung eines Inspirationsdrucks Plnsp verbunden. In Öffnungsstellung des Atemwegeingangsventils 18 ist der Atemweg mit dem Inspirationsdruck Plnsp beaufschlagt. Das zweite Ende 22 ist über ein Atemwegausgangsventil 24 mit einem Atemwegausgangsanschluss der Beatmungsvorrichtung 10 zur Beaufschlagung eines Expirationsdrucks PExp verbunden. In Öffnungsstellung des Atemwegausgangsventils 24 ist der Atemweg mit dem Expirationsdruck PExp beaufschlagt.

Sowohl der Inspirationsdruck Plnsp als auch der Expirationsdruck PExp werden von der Beatmungsvorrichtung 10 nach vorgegebenen zeitlichen Mustern erzeugt, derart dass einzuatmendes Atemgas während einer Inspirationsphase in Richtung der Lunge 28, 30 des Patienten strömt, wie durch den Pfeil 20 in Fig. 1 angedeutet, und während einer Expirationsphase auszuatmendes Atemgas von der Lunge 28, 30 des Patienten zurückströmt, wie durch den Pfeil 26 angedeutet. Normalerweise bleibt während der Inspirationsphase das Atemwegeingangsventil 18 geöffnet und der Atemwegeingang wird mit dem Inspirationsdruck Plnsp - dieser ist in der Regel größer als der Expirationsdruck PExp - beaufschlagt. Während der Expirationsphase ist das Atemwegeingangsventil 18 geschlossen und das Atemwegausgangsventil 24 ist geöffnet. Dann ist der Atemwegeingang mit dem Expirationsdruck PExp beaufschlagt.

Im Zusammenhang mit der vorliegenden Erfindung sind jegliche Formen bekannter Beatmungsmuster einsetzbar, beispielsweise druckkontrollierte Beatmungsformen, volumenkontrollierte Beatmungsformen oder auch Beatmungsformen, bei denen druckkontrollierte und volumenkontrollierte Aspekte kombiniert sind. Neben rein maschinenkontrollierten Beatmungsformen, bei denen der zeitliche Verlauf des Inspirationsdrucks Plnsp und ggf. auch des Expirationsdrucks PExp von der Beatmungsvorrichtung 10 bestimmt werden, sind auch Beatmungsformen denkbar, in denen Spontanatmungsbemühungen des Patienten entweder die maschinelle Beatmung unterstützten können oder die maschinelle Beatmung zur Unterstützung von Spontanatmungsbemühungen des Patienten dient. In solchen Beatmungsformen werden der zeitliche Verlauf von Inspirationsdruck Plnsp bzw. Expirationsdruck PExp sowie häufig ebenfalls die Stellung von Eingangsventil 18 bzw. Ausgangsventil 24 nicht allein von der Beatmungsvorrichtung 10 vorgegeben, sondern von Spontanatmungsbemühungen des Patienten mit beeinflusst.

Die erfindungsgemäß vorgeschlagene Kalibration eines in den Ösophagus einführbaren Osophaguskatheters mit Ballonsonde zur Erfassung eines ösophagealen Drucks, mittels dem auf den transpulmonalen Druck rückgeschlossen werden kann, ist besonders zugeschnitten auf Beatmungsformen, bei denen die Beatmung mittels vollautomatischer Beatmungsmodi erfolgt, beispielsweise bei Beatmung mittels geschlossener Regelkreise, wie sie etwa bei der von der Anmelderin entwickelten Adaptive Support Ventilation (ASV-Beatmung) Anwendung finden. Solche Beatmungsformen zeichnen sich dadurch aus, dass nur minimale manuelle Eingriffe seitens des Bedienpersonals erfolgen müssen und die Beatmungsvorrichtung wichtige Beatmungsparameter wie den positiven endexpiratotischen Druck PEEP oder den maximalen Atemwegsdruck Paw_max im Rahmen vorgegebener Wertebereiche mit Hilfe von geeigneten geschlossenen Regelkreise selbsttätig einstellt bzw. nachstellt.

Das Atemgas kann Umgebungsluft enthalten, wird aber in der Regel einen vorbestimmten Anteil reinen Sauerstoff, im Folgenden als FiO2 bezeichnet, enthalten, der über dem Sauerstoffanteil der Umgebungsluft liegt. Das Atemgas wird in der Regel außerdem befeuchtet sein.

Der Fluss des Atemgases am Atemwegeingang wird mit Hilfe eines Atemwegeingangsflusssensors 36 bestimmt. Der Atemwegeingangsflusssensor 36 beruht auf der Erfassung einer Druckdifferenz dP zwischen einem Eingangsvolumen 38 und einem mit dem Eingangsvolumen 38 in Verbindung stehenden Ausgangsvolumen 40, und liefert eine Bestimmung des Atemgasmassenflusses am Atemwegeingang. Aus dem Drucksignal im Ausgangsvolumen 40 lässt sich zugleich recht einfach der Wert des Atemwegeingangsdrucks Paw ableiten.

Der in den Alveolen der Lunge 28, 30 herrschende Druck ist in Fig. 1 mit Palv angedeutet. Dieser Druck hängt ab vom Atemwegeingangsdruck Paw sowie der Strömung des Atemgases V in die Lunge bzw. aus der Lunge heraus und dem Atemwegswiderstand R. Im Falle eines Druckausgleichs zwischen Atemwegeingang und Alveolen ist der alveoläre Druck Palv gleich dem Atemwegeingangsdruck. Ein derartiger Druckausgleich hat zur Konsequenz, dass der Atemgasfluss V zum Erliegen kommt. Beispielsweise kann ein kurzzeitiges Okklusionsmanöver des Atemwegs, d.h. Atemwegeingangsventil 18 und Atemwegausgangsventil 24 bleiben gleichzeitig geschlossen, zu einem Druckausgleich führen. Dabei muss das Okklusionsmanöver gerade solange dauern, dass der Gasfluss V im Atemweg zum Erliegen kommt. Das sind in der Regel zwischen 1 und 5 s. In diesem Zustand kann dann der alveoläre Druck Palv mittels einer Bestimmung des Atemwegeingangsdrucks Paw bestimmt werden.

Sowohl bei der physiologischen Atmung als auch bei maschineller Beatmung wird der Fluss des Atemgases bestimmt durch eine Druckdifferenz zwischen dem alveolären Druck Palv und dem Atemwegeingangsdruck Paw.

Im Falle der rein physiologischen Atmung wird zum Einatmen eine negative Druckdifferenz, d.h. ein Unterdruck, zwischen dem alveolären Druck Palv und dem Atemwegeingangsdruck Paw erzeugt durch Ausdehnung des Thorax (angedeutet bei 42 in Fig. 1) und damit verbundener Absenkung des Drucks Ppl in dem zwischen Thorax 42 und Lunge 28, 30 gebildeten Pleuraspalt 44. Die Ausatmung erfolgt passiv durch Entspannen des Thorax und elastischer Rückstellung des Lungengewebes. Aus diesem Grund ist bei physiologischer Atmung der Druck im Pleuraspalt Ppl immer kleiner als der alveoläre Druck Palv. Der als Differenz zwischen dem alveolären Druck Palv und dem Druck im Pleuraspalt Ppl definierte transpulmonale Druck Ptp ist damit generell positiv und wird Null im Falle eines vollständigen Druckausgleichs.

Bei maschineller Beatmung wird das Atemgas mit Überdruck in die Lunge gepumpt. Aus diesem Grund ist bei maschineller Beatmung während der Inspirationsphase der Atemwegeingangsdruck Paw = Plnsp größer als der alveoläre Druck Palv und dieser wiederum größer als der Druck im Pleuraspalt Ppl. Aus diesen Druckverhältnissen folgt, dass der transpulmonale Druck Ppl bei maschineller Beatmung während der Inspiration positiv ist. Während der Expiration wird der Atemwegeingang mit einem Atemwegsdruck PExp beaufschlagt, der geringer ist als der alveoläre Druck Palv, so dass Atemgas aus den Alveolen herausströmt. Im Falle eines sehr kleinen Atemwegsdrucks PExp kann es vorkommen, dass am Ende der Expiration, wenn nur noch sehr wenig Gas in der Lunge vorhanden ist, der Druck im Pleuraspalt Ppl den alveolären Druck Palv in so hohem Maße übersteigt, dass ein Teil der Alveolen der Lunge kollabiert. Der transpulmonale Druck Ptp ist dann negativ.

Das Kollabieren der Alveolen lässt sich verhindern, wenn man auch in der Expirationsphase den Atemwegeingang mit einem zusätzlichen positiven Druck beaufschlagt. Es liegt dann am Atemwegeingang dauerhaft, d.h. während der Inspirationsphase und auch während der Expirationsphase ein positiver Atemwegsdruck an. Dieser positive Atemwegsdruck wird als positiver endexpiratorischer Druck oder PEEP bezeichnet.

Der transpulmonale Druck Ptp ist demzufolge eine geeignete Größe zur Einstellung des PEEP. Allerdings ist der transpulmonale Druck Ptp einer unmittelbaren Erfassung nicht zugänglich und lässt sich auch nicht aus den bei maschineller Beatmung regelmäßig erfassten Drücken, wie sie oben beschrieben sind, ermitteln.

In Fig. 1 ist in schematischer Weise eine zusätzliche Ballonsonde 46 zur Messung des als ösophagealer Druck Peso bezeichneten Drucks im Ösophagus (Speiseröhre) 34 angedeutet. Die Ballonsonde 46 hat die Form eines Ballons und ist an einem in den Ösophagus 34 eingeführten Katheter 48 angebracht. Die Ballonsonde 46 liegt innen an der Wand des Ösophagus 34 an und liefert den in auf den Ösophagus 34 am Ort der Ballonsonde 46 einwirkenden Druck. Dieser Druck entspricht bei geeigneter Lagerung des Patienten in guter Näherung dem Druck Ppl im Pleuraspalt. Die beschriebene Ballonsonde 46 zur Erfassung des ösophagealen Drucks Peso und der Umgang mit dieser Sonde ist beispielsweise in Benditt J., Resp. Care, 2005, 50: S. 68-77 beschrieben.

Will man den transpulmonalen Druck Ptp bestimmen, so benötigt man neben dem Druck im Pleuraspalt Ppl noch Information über den alveolären Druck Palv. Eine recht elegante Möglichkeit zur Bestimmung des alveolären Drucks zu einem bestimmten Zeitpunkt t bietet die Erfassung des Atemgasflusses V(t), die mit Hilfe des Flusssensors 36 vorgenommen werden kann. Dann kann man auf den alveolären Druck zum Zeitpunkt t schließen nach der Beziehung: Palv(t) = Paw (t) - R * V(t), wobei R den Atemwegswiderstand bezeichnet. Der Atemwegswiderstand ist für ein und denselben Patienten eine sich im Wesentlichen nicht oder nur vergleichsweise langsam ändernde Größe und kann nach im Stand der Technik bekannten Methoden bestimmt werden. Beispielsweise sei verwiesen auf lotti I.A. et al., Intensive Care Med., 1995, 21: 406-413. Da es zur Bestimmung eines geeigneten PEEP vor allem auf den transpulmonalen Druck am Ende der Expirationsphase Ptp_ee ankommt, wird man im Zusammenhang mit einer automatischen Einstellung des PEEP eine Bestimmung des alveolären Drucks Palv vorzugsweise am Ende der Expirationsphase vornehmen, gemäß der Formel:
Ptp_ee = Palv_ee - Peso_ee = Paw_ee - R * V_ee - Peso_ee.

Der PEEP sollte dann so eingestellt werden, dass Ptp_ee immer positiv bleibt, jedenfalls nie deutlich unter Null sinkt.

Leider lässt die beschriebene Methode der Bestimmung des alveolären Drucks Palv, die recht einfach in einer automatisiert arbeitenden Beatmungseinrichtung 10 realisierbar ist, nur eine vergleichsweise grobe Abschätzung des geeigneten PEEP zu. Das liegt vor allem an dem nur recht ungenau abschätzbaren Atemwegswiderstand R, der zudem in der Regel im zeitlichen Verlauf einer Therapie durchaus einem gewissen Trend unterworfen sein wird.

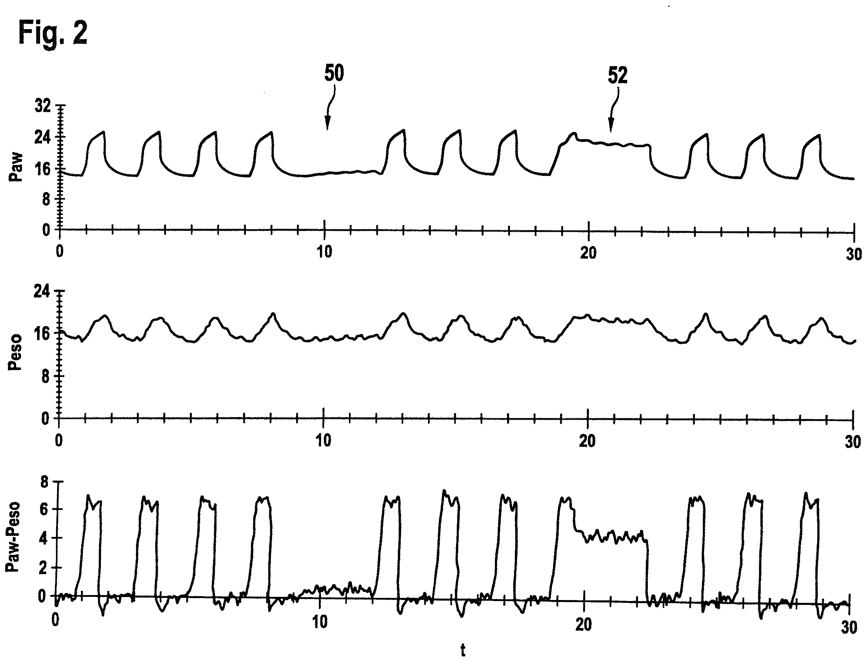

Eine alternative Methode zur Bestimmung des alveolären Drucks Palv beruht auf einem kurzzeitigen Okklusionsmanöver, bei dem sowohl das Atemwegeingangsventil 18 als auch das Atemwegausgangsventil 24 gleichzeitig geschlossen bleiben. In diesem Okklusionszustand tritt ein Ausgleich der im Atemweg herrschenden Drücke ein. Führt man ein solches Okklusionsmanöver am Ende einer Expirationsphase durch, so ist der sich nach einer genügend langen Okklusion im Atemweg einstellende Druck in guter Näherung gleich dem alveolären Druck Palv am Ende der Expirationsphase. Dieser Druck kann dann recht einfach mit Hilfe der am Atemwegeingang angeordneten Drucksonde zur Messung des Atemwegsdrucks Paw erfasst werden. In Fig. 2 ist diese Situation an der mit 50 bezeichneten Stelle dargestellt.

Fig. 2 zeigt übereinander jeweils den zeitlichen Verlauf des Atemwegsdrucks Paw (oben), des mit der Ballonsonde 46 gemessenen ösophagealen Drucks Peso (Mitte) und der Differenz Paw - Peso aus beiden Drücken während mehrerer aufeinander folgender Atemzyklen, zwischen denen auch Okklusionsmanöver durchgeführt wurden. Man kann deutlich die einzelnen Atemzyklen erkennen, mit je einer Inspirationsphase (hoher ansteigender Atemwegsdruck Paw) und einer Expirationsphase (abfallender Atemwegsdruck Paw). Der ösophageale Druck Peso folgt dem Atemwegsdruck Paw, allerdings in abgeschwächter Form. Der in der unteren Kurve dargestellte Differenzdruck Paw - Peso würde - bei genügend langsamer Strömung des Atemgases, so dass immer ein Druckausgleich stattfindet - recht gut dem transpulmonalen Druck Ptp entsprechen. Diese Voraussetzung ist in der Praxis jedoch wegen der sich stark ändernden Atemgasströmung nicht der Fall, abgesehen an den mit 50 und 52 bezeichneten Stellen, bei denen eine kurzzeitige Okklusion am Ende einer Expirationsphase durchgeführt wurde (50, zwischen etwa 8 s und 12 s) bzw. eine kurzzeitige Okklusion am Ende einer Inspirationsphase durchgeführt wurde (52, zwischen etwa 18,5 s und 24,5 s). Die Okklusion dauerte in beiden Fällen etwa 4 s. Das entspricht in dem gewählten Beispiel in etwa der Dauer eines Atemzyklus. Generell sollte die Okklusion so lange dauern, dass ein Druckausgleich im Atemweg stattfindet und der Gasfluss im Atemweg damit zum Erliegen kommt.

Am Ende der mit 50 bezeichneten Stelle des zeitlichen Profils (etwa zwischen 11 s und 12 s, beispielsweise in den letzten ca. 200 ms der Okklusion) entspricht der in der dritten Zeile in Fig. 2 dargestellte Druck Paw - Peso in recht guter Näherung dem transpulmonalen Druck am Ende der Expirationsphase Ptp_ee. Zur Bestimmung von Ptp_ee könnte man beispielsweise den Mittelwert von Paw - Peso über den genannten Zeitraum hinweg bilden. Am Ende der mit 52 bezeichneten Stelle des zeitlichen Profils (etwa zwischen 21,5 s und 22,5 s, beispielsweise in den letzten ca. 200 ms der Okklusion) entspricht der in der dritten Zeile dargestellte Druck Paw - Peso in recht guter Näherung dem transpulmonalen Druck am Ende der Inspirationsphase Ptp_ei. Zur Bestimmung von Ptp_ei könnte man beispielsweise den Mittelwert von Paw - Peso über den genannten Zeitraum hinweg bilden.

Die Bestimmung des transpulmonalen Drucks Ptp mit Hilfe des beschriebenen Ocklusionsmanövers ist genauer als die oben beschriebene Methode mit Hilfe des Atemwegswiderstands R. Allerdings erfordert sie das Durchführen eines Okklusionsmanövers am Ende einer Expirationsphase bzw. am Ende einer Inspirationsphase. Deshalb stört diese Methode naturgemäß den Atemzyklus, und zwar umso erheblicher als die Dauer der Okklusion im Vergleich zur Dauer des Atemzyklus ist. Aus diesem Grund ist es ratsam, so vorzugehen, dass man recht häufig, beispielsweise nach jedem Atemzug oder alle n Atemzüge (n>1), mit Hilfe der Atemwegswiderstandsmethode überprüft, ob ein eingestellter Wert des PEEP und/oder ein eingestellter Wert des maximalen Atemwegsdrucks noch im Rahmen der Vorgaben liegt oder ob ein sich ergebender Wert des transpulmonalen Drucks Ptp_ee noch innerhalb bestimmter Vorgaben für einen normierten transpulmonalen Druck Ptp_ee_ideal liegt. Sollte sich bei dieser Prüfung herausstellen, dass dies nicht der Fall ist und deswegen ein neuer (höherer oder niedrigerer) Wert für den PEEP und/oder den maximalen Atemwegsdruck eingestellt werden sollte, so wird im folgenden Atemzyklus ein Okklusionsmanöver am Ende der Expirationsphase durchgeführt und der neue Wert für den PEEP anhand dieser Okklusion wie oben beschrieben ermittelt. Alternativ könnte man auch das Okklusionsmanöver wie beschrieben alle n Atemzyklen wiederholen, mit n > 1, beispielsweise n = 10, 50, oder 100.

Voraussetzung für die vorangehend beschriebene Bestimmung des transpulmonalen Drucks Ptp mit Hilfe eines Ösophaguskatheters 48 mit Ballonsonde 46 ist, dass zwischen dem transpulmonalen Druck Ptp und dem in der Ballonsonde 46 erfassten ösophagealen Druck Peso eine feste Beziehung besteht. Im Idealfall sollte der in der Ballonsonde 46 erfasste Druck Peso näherungsweise dem Druck im Pleuralspalt Ppl entsprechen, so dass sich der transpulmonale Druck Ptp dann aus der Differenz zwischen Atemwegsdruck Paw und ösophagealem Druck Peso ergibt. In der Praxiskommt es aber regelmäßig vor, dass die Beziehung zwischen dem Druck Peso, der in einem in den Ösophagus 34 eingeführten Katheter 48 mit Ballonsonde 46 gemessen wird, und dem Druck Ppl im Pleuraspalt sich während der Beatmung verändert. Solche Veränderungen können in aller Regel nicht eindeutig auf bestimmte Ursachen oder Ereignisse zurückgeführt werden und treten häufig schleichend auf.

Aus diesem Grund wäre eine Kalibrierung eines solchen Ösophaguskatheters 48 wünschenswert. Allerdings haben sich im Stand der Technik vorgeschlagene Ideen zur Kalibrierung von Ösophaguskathetern in vivo als äußerst empfindlich und zeitraubend erwiesen. Diese Vorschläge sind daher zum Einsatz während der Beatmung von Patienten, insbesondere zur laufenden Überwachung von Ösophaguskathetern auf korrekte Kalibrieung während der Beatmung, nicht besonders geeignet.

Die vorliegende Erfindung stellt ein Kalibriersystem 80 bereit, mit dem eine Verbesserung der Genauigkeit von Druckmesswerten, die von einem in den Ösophagus 34 eingeführten Katheter 48 geliefert werden, erzielt wird. Insbesondere erlaubt das erfindungsgemäße Kalibriersystem 80 eine genauere Wiedergabe des im Pleuraspalt herrschenden Drucks Ppl durch den Ösophaguskatheter 48 und schnellere Reaktion der Messwerte auf sich verändernde Umgebungsbedingungen während andauernder Beatmung. Damit wird eine Kalibrierung des Ösophaguskatheters 48 während der Beatmung möglich, ohne dass die Beatmung dazu unterbrochen werden muss. Dies gilt selbst dann, wenn die Beatmung mittels vollautomatischer Beatmungsmodi erfolgt, beispielsweise bei Beatmung mittels geschlossener Regelkreise, wie bei der von der Anmelderin entwickelten Adaptive Support Ventilation (ASV-Beatmung).

Bei dem erfindungsgemäßen Kalibriersystem 80 ist eine Kalibriersteuerung 60 vorgesehen. Die Kalibriersteuerung 60 ist derart ausgebildet, dass sie die Menge an Messfluid in der Ballonsonde 46 schrittweise verändert, wobei die Kalibriersteuerung 60 bei jeder der auf diese Weise schrittweise als Messpunkte eingestellten Mengen an Messfluid in der Ballonsonde 46 einen ösophagealen Druck Peso aufnimmt und der jeweils eingestellten Menge an Messfluid in der Ballonsonde 46 zuordnet. Hierzu wird das von der Ballonsonde 46 erfasste Drucksignal Peso auch der Kalibriersteuerung 60 übermittelt.

Das Kalibriersystem 80 umfasst eine Pumpanordnung 62 zum Einleiten von Messfluid in die Ballonsonde 46 und Entnehmen von Messfluid aus der Ballonsonde 46 nach Platzieren der Ballonsonde 46 im Ösophagus 34. Die Pumpanordnung 62 weist ein Ventil 64 auf, das in einer mit der Ballonsonde 46 in Fluidverbindung stehenden Förderleitung 66 für Messfluid angeordnet ist. Soweit das Messfluid in der Ballonsonde 46 unter einem Überdruck steht, kann dieses ohne Hilfe der eigentlichen Pumpe einfach durch Ansteuerung des Ventils 64 aus der Ballonsonde 46 entnommen werden.

Das Kalibriersystem 80 weist weiterhin einen Flusssensor 68 auf, insbesondere einen Massenflusssensor, welcher zur Bestimmung einer Menge an in die Ballonsonde 46 eingeleitetem und/oder einer Menge an aus der Ballonsonde 46 entnommenen Messfluid ausgebildet ist. Beispielsweise kann durch Integrieren eines von dem Flusssensor 68 gemessenen Flusses über einen Zeitraum zwischen einem Startzeitpunkt und einem Endzeitpunkt die jeweils in die Ballonsonde 46 eingeleitete bzw. aus der Ballonsonde 46 entnommene Menge an Messfluid bestimmt werden. Der Flusssensor 68 kann beispielsweise so ausgebildet sein, dass er den Massenfluss an Messfluid anhand eines Differenzdrucks bestimmt. In diesem Fall kann der Flusssensor 68 auch zur Erfassung des in der Ballonsonde 46 herrschenden ösophagealen Drucks Peso eingesetzt werden.

Die Kalibriersteuerung 60 kann als eigenständiges Bauteil "in Hardware" realisiert werden. Alternativ kann die Kalibriersteuerung auch als Computerprogrammprodukt, d.h. durch ein entsprechendes Softwareprogramm realisiert werden, das auf einem Prozessor, insbesondere einem Mikroprozessor oder Mikrocontroller, ausgeführt wird. In diesem Fall kann die Software auf einem geeigneten lokalen oder über ein Netzwerk abrufbaren Speichermedium vorgehalten werden. Die Software enthält als Computerprogramm codierte Anweisungen, die dann, wenn die Software in einen Arbeitsspeicher des Prozessors geladen und in Maschinensprache übersetzt wird, den Prozessor dazu veranlasst, die hierin näher beschriebenen Prozeduren auszuführen. Mischformen zwischen Realisierung in Hardware und Realisierung in Software sind selbstverständlich ebenso denkbar. Der Mikroprozessor oder Mikrocontroller kann der Steuerung des Kalibriersystems 80 zugeordnet sein, insbesondere Teil der Steuerung des Kalibriersystems 80 sein.

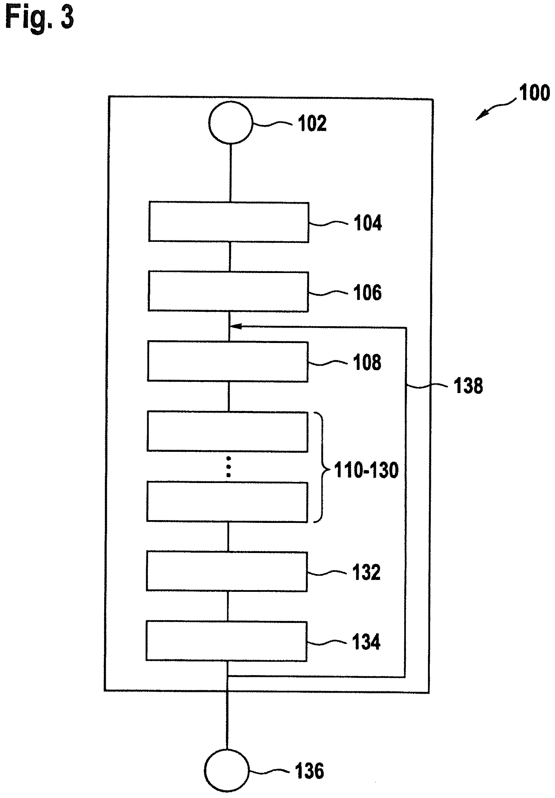

Fig. 3 zeigt anhand eines Flussdiagramms den Ablauf einer Kalibrierung eines Ösophaguskatheters 48 in vivo gemäß einer Ausführungsform der vorliegenden Erfindung.

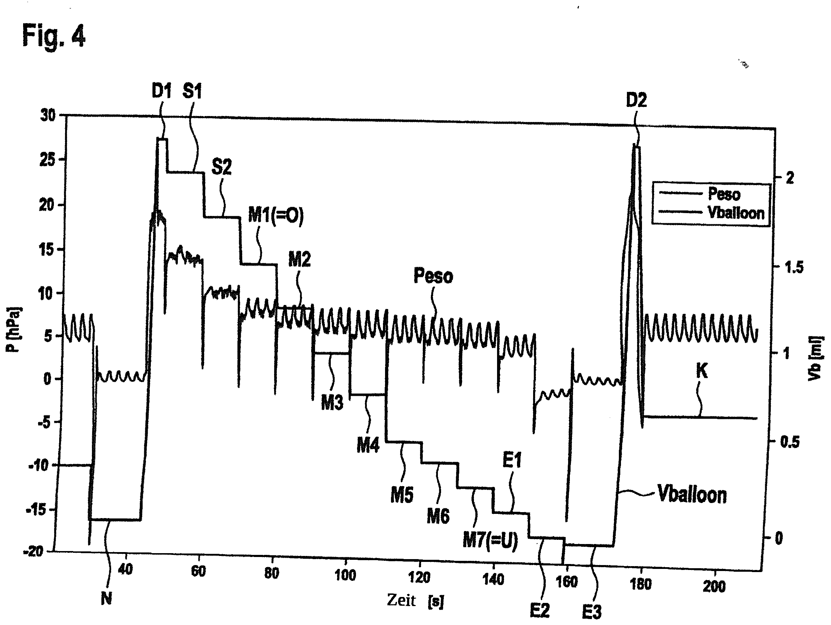

Fig. 4 zeigt schematisch den zeitlichen Verlauf der während einer Kalibrierung gemäß einer Ausführungsform der vorliegenden Erfindung eingestellten Menge an Messfluid Vballoon in der Ballonsonde 46 des Ösophaguskatheters 48 sowie des erfassten Drucks Peso in der Ballonsonde 46 des Ösophaguskatheters 48.

Die Kalibriersteuerung 60 ist derart ausgebildet, dass sie zum Anfahren der jeweiligen Messpunkte S1, S2, M1, M2,..., M7, E1, E2, E3 die Menge an Messfluid Vballoon in der Ballonsonde 46 ausgehend von einem Startwert S2 bis zum Erreichen eines Endwertes E3 in mindestens zwei Schritten in monotoner Weise verändert.

Das Messfluid ist insbesondere Luft.

Die in Fig. 3 gezeigte Kalibrierprozedur 100 beginnt in Schritt 102. Zunächst wird in Schritt 104 Messfluid soweit aus der Ballonsonde 46 abgepumpt, bis in der Ballonsonde 46 ein vorbestimmter Anfangsdruck herrscht. Der in Schritt104 erreichte Zustand ist in Fig. 4 mit "N" bezeichnet.

Danach wird in Schritt 106 wieder Messfluid in die Ballonsonde 46 gepumpt, bis in der Ballonsonde 46 ein vorbestimmter Überdruck herrscht, der oberhalb des erwarteten Messbereichs für den Messzyklus liegt. Der in Schritt104 erreichte Zustand ist in Fig. 4 mit "D1" bezeichnet.

Alle weiteren in den nachfolgenden Schritten 108 bis 130 angefahrenen Messpunkte sind in Fig. 4 mit S1, S2, M1 - M7, E1, E2, E3 bezeichnet.

In dem in Schritt 106 eingestellten Zustand ist die Ballonsonde 46 deutlich überdehnt. Man erkennt das daran, dass in Fig. 4 der Wert des in der Ballonsonde 46 erfassten ösophagealen Drucks Peso deutlich höher liegt als in allen weiteren angefahrenen Messpunkten S1, S2, M1 - M7, E1, E2, E3.

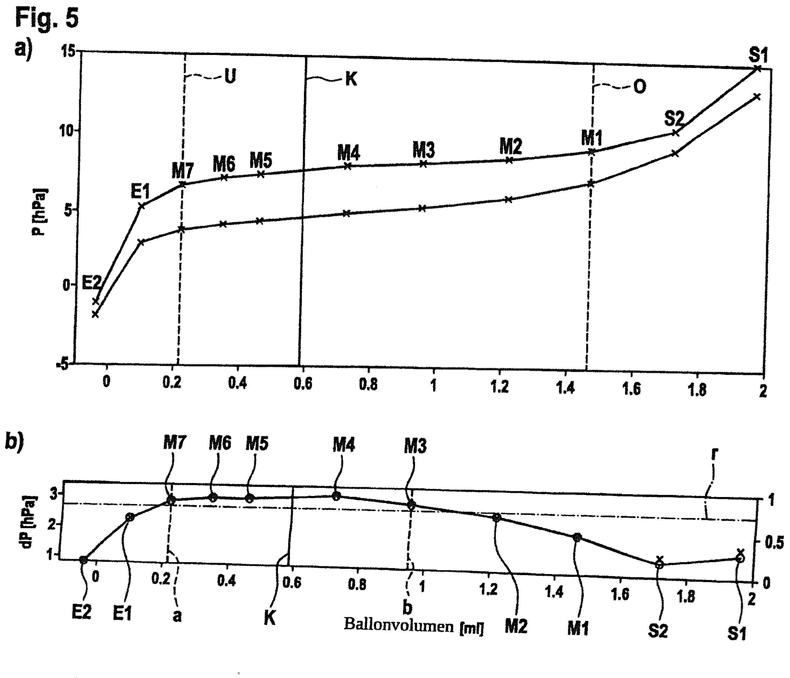

Die Obergrenze des Messbereichs ist in Fig. 4 und 5 mit O bezeichnet und die Untergrenze des Messbereichs ist in Fig. 4 und 5 mit U bezeichnet. Man erkennt, dass bei der in Fig. 4 und 5 gezeigten Kalibrierprozedur die Untergrenze U des Messbereichs bei dem Messpunkt M7 liegt und die Obergrenze O des Messbereichs bei dem Messpunkt M1 liegt. Es wurde für die Obergrenze O und Untergrenze U jeweils die zugehörige Menge Vballoon an Messfluid in der Ballonsonde 46 als Bezugsgröße gewählt. O bezeichnet somit die Menge an Messfluid in der Ballonsonde 46 bei dem Messpunkt M1, der der Obergrenze des Messbereichs entspricht. U bezeichnet die Menge an Messfluid in der Ballonsonde 46 bei dem Messpunkt M7, der der Untergrenze des Messbereichs entspricht.

Ausgehend von Schritt 106 wird in Schritt 108 eine vorbestimmte Menge an Messfluid aus der Ballonsonde 46 abgepumpt. Damit wird eine Menge an Messfluid in der Ballonsonde 46 eingestellt, bei der ein Druck Peso erfasst wird, der ein wenig oberhalb des im Messbereich (siehe Messpunkte M1 bis M7) erwarteten ösophagealen Drucks liegt. Dieser Zustand ist in Fig. 4 und 5 mit "S1" bezeichnet. Alternative könnte auch solange Messfluid aus der Ballonsonde 46 abgepumpt werden, bis ein vorbestimmter Wert von Peso erfasst wird, der ein wenig oberhalb des im Messbereich (siehe Messpunkte M1 bis M7) erwarteten ösophagealen Drucks liegt.

Während des Zustands "S1" wird über eine Mehrzahl von Atemzyklen hinweg der ösophageale Druck Peso in der Ballonsonde 46 erfasst. Man erkennt in Fig. 4, dass der ösophageale Druck Peso die einzelnen Atemzyklen wiedergibt, allerdings nur in einem sehr geringen Ausmaß, was auf geringe Sensitivität der Ballonsonde 46 hindeutet.

Ausgehend von Schritt 108 wird in Schritt 110 eine vorbestimmte Menge an Messfluid aus der Ballonsonde 46 abgepumpt bis der in Fig. 4 und 5 mit "S2" bezeichnete Zustand erreicht wird. Dabei wird in Schritt 110 für den Zustand "S2" dieselbe Prozedur wie in Bezug zu Schritt 108 beschrieben wiederholt. Auch hier erkennt man nur gering ausgeprägte Atemzyklen und damit eine nur geringe Sensitivität der Ballonsonde 46.

Nach Beendigung von Schritt 110 wird in Schritt 112 abermals eine vorbestimmte Menge an Messfluid aus der Ballonsonde 46 abgepumpt, bis der in Fig. 4 und 5 mit "M1" bezeichnete Zustand erreicht wird. Dabei wird in Schritt 112 für den Zustand "M1" dieselbe Prozedur wie in Bezug zu Schritt 108 beschrieben wiederholt.

Man erkennt in Fig. 4 und 5, dass in dem Zustand am Messpunkt M1 (und auch in den nachfolgenden Zuständen an den Messpunkten M2 bis M7) der ösophageale Druck Peso deutlich die einzelnen Atemzyklen wiedergibt. Dabei sind die Maxima der Peso-Kurve den am Ende einer jeweiligen Inspirationsphase erfassten ösophagealen Drücken Peso_insp in der Ballonsonde 46 zuzuordnen und die Minima der Peso-Kurve den am Ende einer jeweiligen Expirationsphase erfassten ösophagealen Drücken Peso_exp in der Ballonsonde 46 zuzuordnen.

Diese Prozedur wird in der Folge (siehe Schritte 114 - 130) mehrmals wiederholt, um weitere Messpunkte M2 - M7, E1, E2 anzufahren. An jedem der weiteren Messpunkte M2 - M7, E1, E2 wird in derselben Weise wie mit Bezug zu Schritt 108, 110 und 112 beschrieben über eine Mehrzahl von Atemzyklen hinweg der ösophageale Druck Peso in der Ballonsonde 46 erfasst und die Maxima der Peso-Kurve den am Ende einer jeweiligen Inspirationsphase erfassten ösophagealen Drücken Peso_insp in der Ballonsonde 46 zugeordnet sowie die Minima der Peso-Kurve den am Ende einer jeweiligen Espirationsphase erfassten ösophagealen Drücken Peso_exp in der Ballonsonde 46 zugeordnet.

Die einzelnen Schritte 110 - 130 sind in dem Flussdiagramm von Fig. 3 nicht im Einzelnen gezeigt.

Anhand von Fig. 4 sieht man, dass diese Schritte jeweils zu einer bestimmten Menge an Messfluid in der Ballonsonde 46 gehören, die als Messpunkte S1, S2, M2 - M7, E1, E2 bezeichnet sind. Die Menge an Messfluid in der Ballonsonde 46 wird beim Übergang von einem der Messpunkte S1, S2, M1 - M7, E1, E2 zu dem nächsten Messpunkt immer in monotoner Weise verändert, insbesondere immer verringert. Dabei kann die Schrittweite, d.h. die Menge, um die die Menge an Messfluid in der Ballonsonde 46 beim Übergang von einem der Messpunkte zum nächsten Messpunkt verändert wird, immer gleich sein. Es ist aber auch denkbar, dass die Menge, um die Menge an Messfluid in der Ballonsonde 46 verändert, in jedem Schritt anders gewählt wird, solange die Veränderung immer in derselben Richtung erfolgt (also die Menge immer verringert wird oder immer vergrößert wird). Da die Zuordnung zwischen einem jeweiligen Messpunkt und der zugehörigen Menge an Messfluid in der Ballonsonde 46 eineindeutig ist, wird der Einfachheit halber im Folgenden nicht nur ein jeweiliger Messpunkt mit D1, S1, S2, M1 - M7, E1, E2 usw. bezeichnet sondern die jeweils zugehörige Menge an Messfluid in der Ballonsonde 46. M1 bezeichnet beispielsweise sowohl den Messpunkt M1 als auch die zugehörige Menge an Messfluid in der Ballonsonde 46. Diese Menge an Messfluid in der Ballonsonde 46 stellt auch die Obergrenze O für den Messbereich dar. In gleicher Weise bezeichnet M7 beispielsweise sowohl den Messpunkt M7 als auch die zugehörige Menge an Messfluid in der Ballonsonde 46. Diese Menge an Messfluid in der Ballonsonde 46 stellt auch die Untergrenze U für den Messbereich dar. Entsprechendes gilt auch für die anderen Messpunkte M2 - M6.

In Fig. 4 erkennt man, dass für die Messpunkte M1 - M7 das erfasste ösophageale Drucksignal Peso deutlich die einzelnen Atemzyklen abbildet, dass aber ab dem Messpunkt E1 das erfasste ösophageale Drucksignal Peso instabil zu werden beginnt. Das deutet an, dass man jetzt in einen Bereich gelangt, in dem die Ballonsonde 46 nur noch unzureichend mit Messfluid befüllt ist und somit auf die von den einzelnen Atemzyklen verursachten Druckänderungen nicht mehr ausreichend reagieren kann. Bei den Messpunkten E2 und E3 kann man davon ausgehen, dass die Ballonsonde 46 vollends kollabiert ist und der ösophageale Druck Peso kaum noch Atemzyklen anzeigt. Der Messzyklus wird dann mit Erreichen von Messpunkt E3 beendet. Messpunkt E3 unterscheidet sich insofern, als bei E3 kein ösophagealer Druck Peso mehr erfasst und zugeordnet wird.

Der Zustand M1 bezeichnet die Obergrenze O des für den Messzyklus nutzbaren Messbereichs. Die eigentliche Kalibration beschränkt sich daher auf diesen durch die Messpunkte M1 - M7 festgelegten Messbereich. Man erkennt in Fig. 4, dass für alle Messpunkte M1 - M7 im Messbereich der in der Ballonsonde erfasste ösophageale Druck Peso im Wesentlichen im gleichen Bereich liegt. Das zeigt an, dass nun die Ballonsonde 46 zusammen mit der Ösophaguswand ein im Wesentlichen elastisches System bildet, das sich entsprechend der Menge an Messfluid in der Ballonsonde 46 dehnt bzw. zusammenzieht. Diese Verhältnisse bleiben gleich, bis mit Erreichen von Messpunkt M7 die Untergrenze U des Messbereichs erreicht ist. Im Messbereich zwischen den Messpunkten M1 und M7, und damit zwischen Werten von der Obergrenze O bis zur Untergrenze U für die Menge an Messfluid in der Ballonsonde 46, besteht eine annähernd lineare Beziehung zwischen der jeweiligen vorbestimmten Menge an Messfluid aus der Ballonsonde 46 und dem jeweils erfassten ösophagealen Druck Peso. Die Steigung einer diese lineare Beziehung wiedergebenden Gerade ist annähernd gleich für die am Beziehung zwischen dem am Ende der Expirationsphase erfassten ösophagealen Druck Peso_ins und der vorbestimmten Menge an Messfluid in der Ballonsonde 46 und für die Beziehung zwischen dem am Ende der Expirationsphase erfassten ösophagealen Druck Peso_exp und der vorbestimmten Menge an Messfluid in der Ballonsonde 46. In den außerhalb des Messbereichs liegenden Bereichen (Messpunkte S1, S2 bzw. Messpunkte E1, E2) verändert sich die Beziehung zwischen der jeweiligen vorbestimmten Menge an Messfluid aus der Ballonsonde 46 und dem jeweils erfassten ösophagealen Druck Peso deutlich. Diese Beziehung ist in diesen Bereichen nicht mehr näherungsweise linear und zeigt einen sehr viel stärkeren Anstieg bzw. Abfall des erfassten ösophagealen Druck Peso mit größerer bzw. kleinerer Menge an Messfluid in der Ballonsonde 46. Auch die Differenz zwischen dem für eine vorbestimmte Menge an Messfluid in der Ballonsonde 46 jeweils am Ende der Expirationsphase erfassten ösophagealen Druck Peso_insp und am Ende der Expirationsphase erfassten ösophagealen Druck Peso_exp wird in den außerhalb des eigentlichen Messbereichs (Messpunkte M1 bis M7) liegenden Bereichen (Messpunkte S1, S2 bzw. E1, E2) sehr schnell kleiner.

Anhand von Fig.5 lässt sich die Bedeutung dieser Kalibrierprozedur erkennen. Fig. 5a zeigt schematisch für jede als Messpunkte S1, S2, M1 - M7, E1, E2 eingestellte Menge an Messfluid Vballoon in der Ballonsonde 46 während eines Messzyklus den jeweils erfassten ösophagealen Druck Peso_insp in der Ballonsonde 46 am Ende der Inspirationsphase und den jeweils erfassten ösophagealen Druck Peso_exp in der Ballonsonde 46 am Ende der Expirationsphase. Fig. 5b zeigt den sich daraus für jeden Messpunkt S1, S2, M1 - M7, E1, E2 jeweils ergebenden Differenzdruck dP zwischen ösophagealem Druck in der Ballonsonde Peso_insp am Ende der Inspirationsphase und ösophagealem Druck in der Ballonsonde Peso_exp am Ende der Expirationsphase.

Man erkennt in Fig. 5, dass innerhalb eines Bereichs zwischen den Messpunkten M1 bis M7 die Veränderung des erfassten ösophagealen Drucks Peso_insp in der Ballonsonde 46 am Ende der Inspirationsphase sowie des erfassten ösophagealen Drucks Peso_exp in der Ballonsonde 46 am Ende der Expirationsphase eine in etwa lineare Beziehung mit der Veränderung der in der Ballonsonde 46 befindlichen Menge Vballoon an Messfluid aufweist. Die Steigung der beiden sich so ergebenden Kurven Peso/Vballoon ist sehr flach und für die ösophagealen Druckwerte am Ende der Inspirationsphase Peso_insp und die ösophagealen Druckwerte am Ende der Expirationsphase Peso_exp im Wesentlichen gleich. Dementsprechend ergibt sich auch, dass die Differenz dP zwischen dem jeweils erfassten ösophagealen Druck Peso_insp in der Ballonsonde 46 am Ende der Inspirationsphase und dem jeweils erfassten ösophagealen Druck Peso_exp in der Ballonsonde 46 am Ende der Expirationsphase in dem durch die Messpunkte M1 bis M7 definierten Messbereich im Wesentlichen konstant bleibt, während diese Differenz dP in den außerhalb dieses Messbereichs liegenden Bereichen, in denen die Messpunkte S1, S2 und E1, E2 liegen, schnell sehr klein wird. Daraus ergibt sich, dass eine optimale Kalibration des Ösophaguskatheters 48 bei einer Menge an Messfluid in der Ballonsonde 46 liegt, die innerhalb des durch die Messpunkte M1 bis M7 definierten Messbereichs liegt, also innerhalb des Bereichs an Menge von Messfluid in der Ballonsonde 46, zwischen den beiden in Fig. 5a) eingezeichneten vertikalen gepunkteten Linien O und U liegt. Dementsprechend wählt die Kalibriersteuerung 60 als Ergebnis der Kalibrierung eine Menge an Messfluid in der Ballonsonde 46 aus, die innerhalb des durch die Messpunkte M1 bis M7 definierten Messbereichs liegt. Diese Menge ist durch die vertikale Linie K in Fig. 5b) wiedergegeben und wird nach Beendigung der Kalibrierprozedur als Menge an Fluid in der Ballonsonde 46 eingestellt, wie in dem mit K bezeichneten horizontal verlaufenden Teil der Vballoon-Kurve im rechten Teil der Fig. 4 zu sehen ist.

Fig. 5b) zeigt, wie die Auswahl der optimalen Menge an Messfluid in der Ballonsonde 46 innerhalb des durch die Messpunkte M1 bis M7 definierten Messbereichs abläuft. Innerhalb des Messbereichs wird zunächst derjenige Messpunkt gesucht, bei dem die Differenz dP zwischen dem jeweils erfassten ösophagealen Druck Peso_insp in der Ballonsonde 46 am Ende der Inspirationsphase und dem jeweils erfassten ösophagealen Druck Peso_exp in der Ballonsonde 46 am Ende der Expirationsphase maximal ist. In dem in Fig. 5b) gezeigten Beispiel ist das bei dem Messpunkt M4 der Fall. Danach wird eine erlaubte Schwankungsbreite um diese maximale Differenz dPmax festgelegt. Innerhalb dieser Schwankungsbreite liegende Werte der Differenz dP werden als nicht signifikant verschieden von dem Maximalwert dPmax angesehen. In dem in Fig. 5b) gezeigten Beispiel beträgt die erlaubte Schwankungsbreite 10 % der beim Messpunkt M4 ermittelten maximalen Differenz dPmax. Diese Schwankungsbreite ist durch die strichpunktierte Linie r in Fig. 5b) angedeutet. Innerhalb der Schwankungsbreite liegen die für die Messpunkte M3, M4, M5, M6 und M7 ermittelten Differenzen dP. Alle diese Messpunkte liegen benachbart zum Messpunkt M4 und benachbart zueinander. Daher wird ein Bereich an Menge von Messfluid in der Ballonsonde 46, der zwischen den Messpunkten M3 und M7 liegt, als gleichwertig hinsichtlich Befüllung der Ballonsonde 46 mit Messfluid angesehen. Dieser Bereich ist in Fig. 5b) durch vertikale gestrichelte Linien a und b bezeichnet. Als optimale Menge an Messfluid in der Ballonsonde 46 wird die Mitte des zwischen den Linien a und b liegenden Bereichs gewählt. Auf diese Weise erhält man die optimale Menge an Messfluid in der Ballonsonde 46 als Ergebnis der Kalibrierung, siehe die vertikale Linie K in Fig. 5b). Diese Menge K wird nach Beendigung der Kalibrierprozedur als Menge an Fluid in der Ballonsonde 46 eingestellt, siehe den Teil der Vballoon-Kurve ganz rechts in Fig. 4.

Nach Beendigung der Kalibrierprozedur leitet das Kalibriersystem 80 in Schritt 132 wieder eine Menge an Messfluid in die Ballonsonde, die deutlich oberhalb der den Messpunkten M1 bis M7 des Messbereichs entsprechenden Menge liegt. Auch in diesem Schritt soll wieder eine Überdehnung der Ballonsonde 46 herbeigeführt werden (in Fig. 4 ist dieser Zustand mit "D2" bezeichnet), bevor dann in Schritt 134 durch Entnehmen einer entsprechenden Menge an Messfluid aus der Ballonsonde 46 der in der vorangehenden Kalibrieprozedur ermittelte kalibrierte Zustand eingestellt wird, der in Fig. 4 mit "K" bezeichnet ist (und in dem demzufolge die Ballonsonde 46 mit der optimalen Menge K an Messfluid gefüllt ist).

Alternativ könnte auch vorgesehen sein, dass sich an den in den Fig. 3 bis 5 dargestellten Messzyklus noch ein weiterer Messzyklus (oder mehrere weitere Messzyklen) anschließt, bevor der kalibrierte Zustand K eingestellt wird. In einem solchen Fall geht die Prozedur von Schritt 134 zurück zu Schritt 108 und wiederholt die Schritte für den weiteren Messzyklus analog zu den Schritten 110 - 130 für den ersten Messzyklus. In Fig. 3 ist dies durch die Linie 138 angedeutet.

Da die Kalibriersteuerung 60 zum Anfahren der jeweiligen Messpunkte S1, S2, M1 - M7, E1, E2 die Menge an Messfluid in der Ballonsonde 46 ausgehend von einem Startwert S1 bis zum Erreichen eines Endwertes E3 in mindestens zwei Schritten in monotoner Weise verändert, lässt sich die Kalibrierung in kurzer Zeit, beispielsweise innerhalb von nur wenigen Minuten, erledigen. Dies erlaubt es, die Kalibrierung während einer laufenden Beatmung von Zeit zu Zeit zu wiederholen und dadurch sicherzustellen, dass der Ösophaguskatheter 48 immer korrekt kalibriert ist, auch wenn sich im Laufe der Beatmung die optimale Befüllung der Ballonsonde 46 verändert. Das macht es möglich, Patienten über längere Zeiträume hinweg in automatisierten Beatmungsmodi zu beatmen.

## Patentansprüche

1. Kalibriersystem (60) zur automatisierten Einstellung einer betriebsgemäßen Befüllung eines in den Ösophagus (34) einführbaren Ösophaguskatheters (48) mit Ballonsonde (46) zur Bestimmung eines ösophagealen Drucks (Peso), insbesondere für eine Beatmungsvorrichtung (10), umfassend:
- eine Anordnung zum Befüllen der Ballonsonde (46) mit einem Messfluid nach Platzieren der Ballonsonde (46) im Ösophagus (34),
- einen Drucksensor zum Erfassen des in der Ballonsonde (46) herrschenden ösophagealen Drucks (Peso), sowie
- eine Kalibriersteuerung (60) die derart ausgebildet ist, dass sie die Menge an Messfluid in der Ballonsonde (46) schrittweise verändert, wobei die Kalibriersteuerung (60) bei jeder der auf diese Weise schrittweise als Messpunkte (S1, S2, M1 - M7, E1, E2) eingestellten Mengen an Messfluid in der Ballonsonde (46) einen von dem Drucksensor erfassten ösophagealen Druck (Peso) aufnimmt und der jeweils eingestellten Menge an Messfluid in der Ballonsonde (46) zuordnet,
wobei die Kalibriersteuerung (60) derart ausgebildet ist, dass sie zum Anfahren der jeweiligen Messpunkte (S1, S2, M1 - M7, E1, E2) die Menge an Messfluid ausgehend von einem Startwert bis zum Erreichen eines Endwertes in mindestens zwei Schritten in monotoner Weise verändert.

2. Kalibriersystem (80) nach Anspruch 1, ferner umfassend eine Fluidablasseinrichtung, die zum Ablassen von Messfluid aus der Ballonsonde (46) ausgebildet ist,
wobei die Kalibriersteuerung (60) die Ablasseinrichtung zum Anfahren der jeweiligen Messpunkte (S1, S2, M1 - M7, E1, E2) derart ansteuert, dass die Menge an Messfluid in der Ballonsonde (46) ausgehend von dem Startwert bis zum Erreichen des Endwertes in mindestens zwei Schritten in monotoner Weise abnimmt,
wobei die Kalibriersteuerung (60) insbesondere derart ausgebildet ist, dass sie die Anordnung zum Befüllen der Ballonsonde (46) mit einem Messfluid wenigstens in einem ersten Messzyklus zum Befüllen der Ballonsonde (46) mit einer Menge an Messfluid ansteuert, die größer ist als eine obere Grenze des Messbereichs zwischen Startwert und Endwert.

3. Kalbriersystem (80) nach Anspruch 2, wobei die Kalibriersteuerung (60) beim Anfahren der jeweiligen Messpunkte (S1, S2, M1 - M7, E1, E2) die Ablasseinrichtung derart ansteuert, dass die Menge an Messfluid in der Ballonsonde (46) ausgehend von dem Startwert schrittweise immer weiter verringert wird, bis bei Erreichen oder Unterschreiten eines vorgegebenen minimalen endexpiratorischen ösophagealen Druckes (Peso), beispielsweise - 5 hPa, der Endwert erreicht ist.

4. Kalibriersystem (80) nach einem der Ansprüche 1 bis 3, wobei die Kalibriersteuerung (60) dazu ausgebildet ist, mindestens zwei Messzyklen nacheinander auszuführen,
wobei insbesondere der Messbereich der mindestens zwei aufeinanderfolgenden Messzyklen verschieden ist, wobei insbesondere ein vorangehender Messzyklus den Messbereich für einen nachfolgenden Messzyklus festlegt.

5. Kalibriersystem (80) nach Anspruch 4, wobei die Kalibriersteuerung (60) derart ausgebildet ist, dass sie den Abstand zwischen aufeinanderfolgenden Messpunkten für den vorangehenden Messzyklus und für den nachfolgenden Messzyklus unterschiedlich einstellt.

6. Kalibriersystem (80) nach einem der Ansprüche 1 bis 5, wobei die Kalibriersteuerung (60) dazu ausgebildet ist, die Schrittweite zwischen aufeinander folgenden Messpunkten innerhalb des Messbereichs in einem Messzyklus in adaptiver Weise zu bestimmen.

7. Kalibriersystem (80) nach einem der Ansprüche 4 bis 6, wobei die Kalibriersteuerung (60) derart ausgebildet ist, dass zwischen einem vorangehenden Messzyklus und einem nachfolgenden Messzyklus keine vollständige Entleerung des Messfluids aus der Ballonsonde (46) stattfindet.

8. Kalibriersystem (80) nach einem der Ansprüche 1 bis 7, wobei die Kalibriersteuerung (60) derart ausgebildet ist, dass sie für jeden Messpunkt (S1, S2, M1 - M7, E1, E2), d.h. für jede eingestellte Menge an Messfluid in der Ballonsonde (46), zwischen dem Startwert und dem Endwert je einen Messwert für den ösophagealen Druck (Peso_insp) am Ende einer Inspirationsphase und einen Messwert für den ösophagealen Druck (Peso_exp) am Ende einer Expirationsphase ermittelt, und dann die Differenz zwischen dem ösophagealen Druck (Peso_insp) am Ende der Inspirationsphase und dem ösophagealen Druck (Peso_exp) am Ende der Expirationsphase bestimmt,
wobei die Kalibriersteuerung (60) insbesondere derart ausgebildet ist, dass sie innerhalb eines zwischen dem Startwert für die Menge an Messfluid in der Ballonsonde (46) und dem Endwert für die Menge an Messfluid in der Ballonsonde (46) liegenden Bereichs einen Maximalwert für die Differenz zwischen dem ösophagealen Druck (Peso_insp) am Ende der Inspirationsphase und dem ösophagealen Druck (Peso_exp) am Ende der Expirationsphase bestimmt.

9. Kalibriersystem (80) nach Anspruch 8, wobei die Kalibriersteuerung (60) derart ausgebildet ist, dass sie für einen jeweiligen Messpunkt zwischen dem Startwert und dem Endwert den Messwert für den ösophagealen Druck (Peso_insp) am Ende einer Inspirationsphase und den Messwert für den ösophagealen Druck (Peso_exp) am Ende einer Expirationsphase bei weiterlaufender Beatmung ermittelt,
wobei die Kalibriersteuerung (60) insbesondere derart ausgebildet ist, dass sie die für die jeweiligen Messpunkte (S1, S2, M1 - M7, E1, E2) ermittelten Differenzen zwischen dem ösophagealen Druck (Peso_insp) am Ende der Inspirationsphase und dem ösophagealen Druck (Peso_exp) am Ende der Expirationsphase miteinander vergleicht, und dann, wenn über eine Mehrzahl von aufeinander folgenden Messpunkten die jeweils ermittelte Differenz innerhalb einer vorgegebenen Schwankungsbreite um den Maximalwert liegt, eine optimale Menge an Messfluid in der Ballonsonde (46) als eine Menge mit vorgegebenen Abstand zu einem oberen und/oder unteren Rand dieser Mehrzahl aufeinander folgender Messpunkte bestimmt.

10. Kalibriersystem (80) nach einem der Ansprüche 1 bis 9, wobei die Kalibriersteuerung (60) derart ausgebildet ist, dass sie für jeden Messpunkt (S1, S2, M1 - M7, E1, E2) zwischen dem Startwert und dem Endwert eine Mehrzahl von Messwerten, insbesondere eine Mehrzahl von Messwertpaaren für den ösophagealen Druck (Peso_insp) am Ende einer Inspirationsphase und für den ösophagealen Druck (Peso_exp) am Ende einer Expirationsphase, ermittelt, wobei die Kalibriersteuerung (60) anhand der Mehrzahl von Messwerten bzw. Messwertpaaren für jeden Messpunkt (S1, S2, M1 - M7, E1, E2) einen Durchschnitt und eine statistische Streuung für den Messwert bzw. die Messwertpaare, oder eine daraus abgeleitete Größe, insbesondere die Differenz zwischen dem ösophagealen Druck (Peso_insp) am Ende der Inspirationsphase und dem ösophagealen Druck (Peso_exp) am Ende der Expirationsphase, bestimmt und die Anzahl von Messungen je Messpunkt (S1, S2, M1 - M7, E1, E2) so festlegt, dass der erhaltene Durchschnitt als statistisch signifikant angesehen werden kann.

11. Kalibriersystem (80) nach einem der Ansprüche 1 bis 10, wobei die Kalibriersteuerung (60) derart ausgebildet ist, dass sie für jeden Messpunkt (S1, S2, M1 - M7, E1, E2) zwischen dem Startwert und dem Endwert überwacht, ob die jeweilige Messung des ösophagealen Drucks (Peso) durch äußere Umstände beeinträchtigt ist, und die jeweilige Messung verwirft, wenn solche äußeren Umstände festgestellt werden.

12. Kalibriersystem (80) nach einem der Ansprüche 1 bis 11, wobei die Kalibriersteuerung (60) dazu ausgebildet ist, anhand der während der Kalibrierprozedur ermittelten Daten einen Qualitätsindex zu berechnen.

13. Kalibriersystem (80) nach einem der Ansprüche 1 bis 12, wobei die Kalibriersteuerung (60) derart ausgebildet ist, das der ösophageale Druck (Peso) einen vorgegebenen Maximaldruck nicht überschreitet.

14. Verfahren zur automatisierten Kalibrierung einer betriebsgemäßen Befüllung eines in den Ösophagus (34) einführbaren Ösophaguskatheters (48) mit Ballonsonde (46) zur Bestimmung eines ösophagealen Drucks (Peso), insbesondere für eine Beatmungsvorrichtung (10), umfassend folgende Schritte:
- Befüllen der Ballonsonde (46) mit einem Messfluid nach Platzieren der Ballonsonde (46) im Ösophagus(34),
- Erfassen des in der Ballonsonde (46) herrschenden ösophagealen Drucks (46), sowie
- Schrittweises Verändern einer Menge an Messfluid in der Ballonsonde (46), wobei bei jeder der auf diese Weise schrittweise als Messpunkte (S1, S2, M1 - M7, E1, E2) eingestellten Mengen an Messfluid in der Ballonsonde (46) der ösophageale Druck (Peso) erfasst und der jeweils eingestellten Menge an Messfluid in der Ballonsonde (46) zuordnet, wird wobei zum Anfahren der jeweiligen Messpunkte (S1, S2, M1 - M7, E1, E2) die Menge an Messfluid ausgehend von einem Startwert bis zum Erreichen eines Endwertes in mindestens zwei Schritten in monotoner Weise verändert wird,
wobei das Verfahren insbesondere wenigstens einen weiteren der in den Ansprüchen 1 bis 13 mit Bezug auf eine Ausbildung eines Kalibriersystems (80) genannten Verfahrensschritte umfasst.

15. Computerprogrammprodukt, welches Programmanweisungen enthält, bei deren Ausführung auf einer Datenverarbeitungsanlage, insbesondere auf einem Mikroprozessor oder einem Mikrocontroller zur Steuerung eines Ösophaguskatheters (48) mit Ballonsonde (46), ein Verfahren nach Anspruch 14 ausgeführt wird.
